(19) Europäisches Patentamt
European Patent Office
Office européen des brevets

(11) Publication number: 0 192 786 B1

(12) EUROPEAN PATENT SPECIFICATION

(45) Date of publication of patent specification: 07.02.90

(51) Int. Cl.⁵: A 61 M 5/14

(21) Application number: 85101770.7

(22) Date of filing: 18.02.85

# (54) Processor-controlled angiographic injector device.

(43) Date of publication of application:
03.09.86 Bulletin 86/36

(45) Publication of the grant of the patent:
07.02.90 Bulletin 90/06

(84) Designated Contracting States:
AT BE CH DE FR GB IT LI LU NL SE

(56) References cited:
GB-A-2 009 453
US-A-4 006 736
US-A-4 273 122
US-A-4 470 758

(73) Proprietor: Medrad Incorporated
566 Alpha Drive
Pittsburgh Pennsylvania 15238 (US)

(72) Inventor: Reilly, David M.
2318 Middle Road
Glenshaw Pennsylvania 15116 (US)
Inventor: Potter, Ross H.
658 13th Street
Oakmont Pennsylvania 15139 (US)
Inventor: Stulen, John A.
260 Crestmont Road
Pittsburgh Pennsylvania 15237 (US)
Inventor: Toft, Eric M.
5747 Woodmont
Pittsburgh, Pennsylvania 15217 (US)
Inventor: Hirschman, Alan D.
101 Candlewiek Drive
Glenshaw Pennsylvania 15116 (US)
Inventor: Clark, John G.
207 Irene Street
Pittsburgh, PA 15223 (US)

(74) Representative: Lorenz, Eduard
Rechtsanwälte Eduard Lorenz - Bernhard Seidler
Margrit Seidler - Dipl.-Ing. Hans-K. Gossel Dr. Ina
Philipps - Dr. Paul B. Schäuble Dr. Siegfried
Jackermeier
Widenmayerstrasse 23 D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 192 786 B1

**Description**

Background of the invention

This invention pertains to angiographic devices for injecting into a patient contrast media at a controlled rate and pressure during x-ray photography. More specifically, this invention is an improvement over US 4 006 736, which improvement concerns automated control of such angiographic injector devices being responsive to input information supplied by an operator to develop control signals for automatically controlling the injection process.

An angiographic injector device according to the preamble part of claim 1 is known from US 4 006 736.

An angiographic injector is useful for controlling the delivery rate, amount, duration, and pressure of contrast media, usually a liquid iodine solution, injected into a patient. Such devices are used in x-ray photography to enhance the contrast of the image obtained thereby. In a typical operation of such device, an operator loads the same with a certain amount of contrast media, connects a delivery tube extending from a fluid reservoir of the device to a catheter placed in the vascular system of a patient, and then actuates the device by forcing the media into the blood stream while exposing the patient to x-rays during the photographic process. Among other things, it is very important that the proper amount of contrast media, as well as the pressure and rate at which it is delivered, be controlled accurately for safe and desirable results.

One such angiographic injector device over which the present invention is an improvement is described in U.S. Patent No. 4,006,736 issued to Kranys et al, commonly owned by the assignee hereof. As with many other types of angiographic injector devices, this system is rather mechanical and although efficient, does not take advantage of certain potential automatic control and test features which can facilitate its use and reduce the likelihood of errors during its operation. As known, certain errors can be fatal or expose the patient to undue risks of harm.

As examples of potential capabilities under automated control, it is often desirable to provide multi-level injections during an x-ray photographing sequence. In this case, the contrast media is injected, for example, in step-wise changing flow rates and/or pressures. It is also desirable to provide multiphasic injections in which the programmed injection profile is delivered several times in succession under operator or remote control. Also, it may be desirable to automatically compute parameters involved in the injection of a specific amount of contrast media and to enable the size of the syringe to be changed without adversely affecting the operation of the injection and without requiring reprogramming. Such features are not known to exist with prior art devices. Moreover, certain mechanical control and electronic control features can be integrated to enhance reliability, such as by providing a mechanical stop member to prevent further movement of a syringe plunger when a predetermined amount of contrast media already has been injected.

Additionally, rather than requiring the operator to calculate flow rates and/or volumes, this can automatically be computed by a processor controlled system as a function of an injection parameter supplied to the system by the operator, whereupon the system itself would then calculate the corresponding pressure and control signals for delivery of the media. Still further, the use of a microprocessor in an angiographic injector device enables various programming verification steps not otherwise available. These are only a few automatic control features which are not known to exist with prior art systems.

In view of the foregoing, it is the object of the present invention to provide a processor-controlled angiographic injector device for automatically delivering contrast media at controlled rates, pressures or volumes, which rates and pressures or volumes are automatically calculated on the basis of injection parameters supplied thereto by a user.

This object is accomplished by the features of the caracterizing part of claim 1. Preferred embodiments of the invention are described in the sub-claims.

Furthermore, the device according to the invention is able to provide safety and/or control features which limit the injection pressure and/or delivery of contrast media when ceratin limits are exceeded, such as a pressure limit of contrast media in the syringe.

The invention is also able to provide a mechanical stop mechanism for mechanically preventing the plunger of a syringe from further movement when a given amount of contrast media has been injected, wherein the stop position is automatically determined on the basis of injection information supplied to the device.

It is also possible in the present invention to interlock the operation of the mechanical stop mechanism and a plunger drive circuit of the injector device so that they alternate in operation to ensure that the drive circuit of the plunger does not operate until the drive circuit of the stop mechanism has completed its setting of the stop position.

The invention is furthermore able to provide a multi-level injection sequence under processor control whereby the duration and/or injection rate and/or pressure may step-wise be changed during separate injection sequences, as well as to provide means for compensating the plunger drive rate and delivered pressure on the basis of syringe size during the injection process.

Additionally the present invention provides preprogrammed injection parameters stored in a memory which may conveniently be recalled instantly and to provide means for retaining the stored parameters in the memory in the event of power interruption to the device.

Another advantage of the present invention is to provide a plurality of reliability features, such as parameter verification, self-calibration, and self-testing of the various components of the device in order to improve safety.

A yet further feature of the present invention is to provide means for providing messages from the angiographic injector to the operation, in human readable format.

Summary of the invention

To attain the foregoing and additional objects and advantages, the invention comprises a processor-controlled angiographic injector device including a pressure jacket for receiving a syringe containing liquid contrast media, contrast media drive means for forcing the media from the syringe, and processor control system for calculating injection control signals and for controlling the delivery of the contrast media. The processor control system is programmed to elicit injection parameters from an operator or from a pre-programmed storage module and, on the basis of the injection parameters, for calculating the necessary control signals for a closed-loop servo system which actuates the plunger of a syringe. The processor also determines position limits of plunger actuation and effects actuation of a mechanical stop member to block further movement of the plunger when a predetermined volume of media is injected. To improve reliability, actuation of motors for the mechanical stop member and the plunger drive are alternately enabled by a safety relay.

In alternative embodiments of the invention, a preprogrammed storage module is provided for storing routine injection parameters so that they can be instantly recalled. A hardware verification system comprising primary and secondary memories for storing duplicates of injection parameters also is provided. The controller additionally provides multi-phasic injection sequences which involve step-wise changing of the injection parameters (e.g., rate, duration, and/or pressure) during an injection sequence.

Further, the angiographic injector device includes a watchdog circuit which monitors failures in the processor-controlled system, such as in the memories, and the processor itself, and in response to a failure, inhibits the plunger drive means. A self-check feature periodically performs diagnostic routines and a self-calibration feature re-calibrates the servo positioning system when there is a deviation from desired accuracy. To aid in certain types of x-ray photography, such as arteriography or ventriculography, an alternative embodiment of the device includes means for monitoring an ECG waveform and for injecting a small bolus of contrast media at a given rate and pressure during the diastolic interval. Moreover, the alternative embodiment includes an interface circuit for providing external digital communication for transferring either status and/or control information (e.g. injection parameters).

These and other embodiments, aspects, advantages and features of the invention will become apparent upon review of the succeeding disclosure taken in connection with the accompanying drawings. The invention, though, is pointed out particularly by the appended claims.

Brief description of the drawings

Figure 1A depicts a block circuit diagram of a preferred embodiment of the invention.

Figure 1B depicts a watchdog timer circuit of Figure 1A for monitoring CPU failures.

Figure 2 depicts circuit components of the servo amplifier of Figure 1.

Figure 3 depicts a functional block diagram of the servo control unit of Figure 1.

Figure 4 depicts a circuit diagram of the mechanical stop controller of the inventive angiographic device of Figure 1.

Figures 5A is a daigram of the mechanical stop mechanism which responds to the controller of Fig. 4 of the angiographic injector device.

Figure 5B depicts an arrangement for visually indicating a scaled volume of contrast media ejected from a syringe.

Figure 6 depicts the front control and display panel of Figure 1.

Figures 7A, 7B, 7C and 7D depict the sequence of the self-test and self-calibrating features under which the automated injector device goes.

Figures 8A, 8B, 8C and 8D show flow diagrams of the operation of the processor controlled injection procedures.

Detailed description of illustrative embodiment

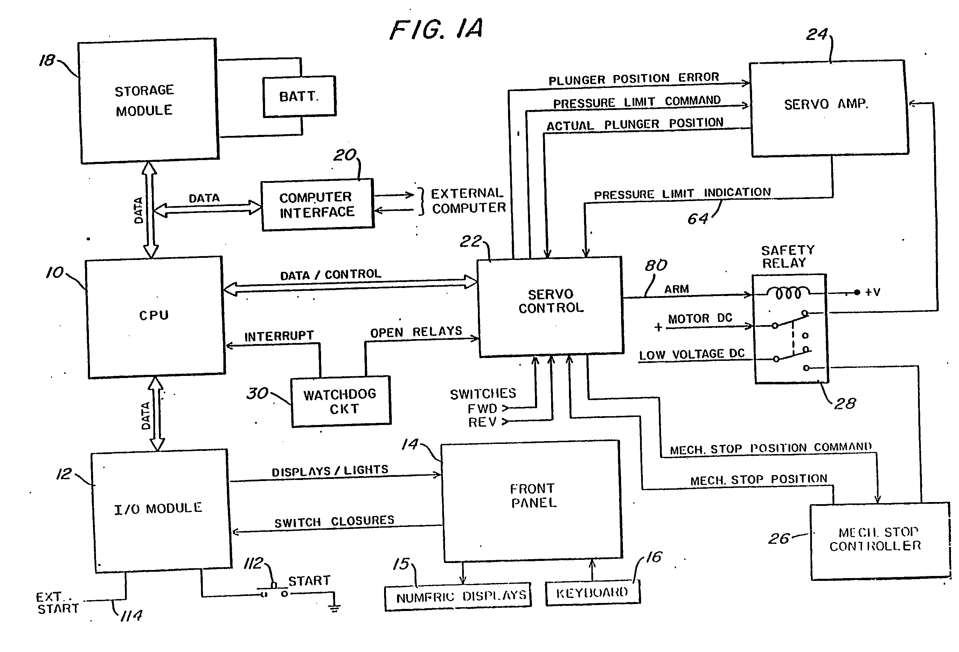

Referring to Figure 1A, primary functions of the automated angiographic injector device are controlled, monitored and executed by a central processing unit (CPU) 10 such as a commercially known Z80A microprocessor which includes a memory. The memory comprises a read only portion (ROM) such as a 2732 EPROM and a random access portion (RAM) such as a 4016 static NMOS RAM coupled to the CPU 10 is an I/O module 12 which, under control the CPU 10, prompts the operator for certain input parameters and also alerts the operator of error conditions in the system. The I/O module is composed of peripheral devices such as the Zilog Z80-PIO peripheral input/output controller. An indicator panel 14, and keyboard 16 provides operator/injector device interface. In practice, the panel 14 includes a flat sealed membrane to shield electrical switch contacts from contamination. A storage module 18 stores pre-programmed injection parameters which may be instantly recalled and supplied to the CPU 10 when a routine injection procedure is to be performed. The injection module 18 comprises two primary random access memories. Each memory contains a duplicate of the information of the other memory, and prior to an injection procedure, the contents thereof are compared for consistency. If inconsistent, the injection is inhibited.

A computer interface 20 provides external remote communication with the automated injector device and functions to transfer both status information and control information with the device for remote operation and/or monitoring to provide communication compatibility with various external devices. The interface 20 includes, but is not limited to, a standard universal asynchronous receive/transmit port connectible by way of a commercially known RS-232 serial channel or other parallel interface.

The contrast media to be injected into the patient is normally contained in a syringe, the plunger thereof being actuated to force the media therefrom into the vascular system of a patient through a catheter. The delivery rate and volume are normally derived from position signals indicative of the plunger position. Pressure is derived from current supplied to the motor. A servo control network 22 applies a conventional error signal to a servo system for controlling the position of the syringe plunger to control the flow rate and pressure of the contrast media. More specifically, the servo control network 22 supplies error signals to a servo amplifier 24 which energizes a conventional D.C. motor for controlling the position of the plunger.

Also provided for safety enhancement is a mechanical stop controller 26 which, under the control of the CPU 10 and an interlock safety relay 28, automatically positions a mechanical stop member (subsequently described) under control of the CPU 10 to mechanically stop further plunger movement thus preventing additional injection after the desired volume of contrast media has been injected. The safety relay 28 alternately enables the mechanical stop motor and the plunger motor so that one and only one may be operative at a given time. This feature enhances reliability because it ensures that the mechanical stop fully reaches its appropriate position before it is possible to drive of the syringe plunger.

A watchdog circuit 30 monitors certain functions of the CPU 10 to effect shutdown of the system by inhibiting delivery of contrast media in the event of a failure or a fault. The watchdog circuit, as shown in Figure 1B, consists of a retriggerable timer (such as a 74123 monostable multivibrator) which generates a pulse after a fixed time interval unless a strobe signal is received from a data selector 13 before the interval elapses. Under normal operating condition, the CPU 10 executes its control sequence and periodically outputs an address to the data selector 13 (such as 74154) which generates the strobe signal. If a CPU or memory failure occurs, the normal program sequence is interrupted, the data selector 13 is not addressed, the strobe signal is not generated, and the timer 11 is not retriggered. The resulting pulse from the timer automatically opens the safe relay, thereby removing power from the plunger and forcing the processor to execute a non-maskable interrupt (NMI). The NMI forces the CPU 10 to inform the user of a fault condition and then to halt.

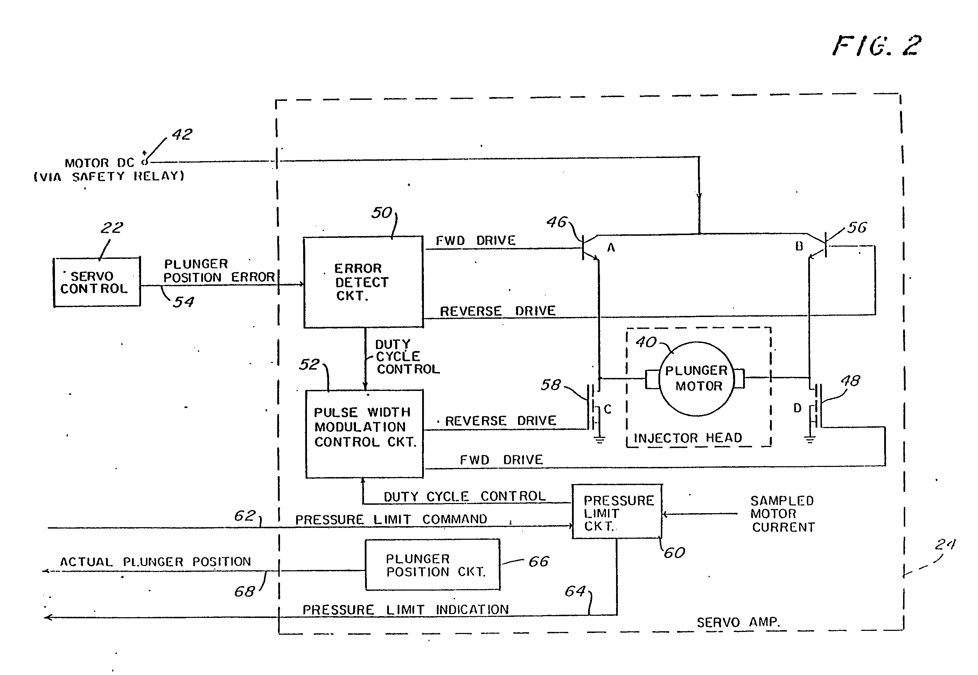

Figure 2 depicts the servo amplifier 24 of Figure 1A. The servo amplifier provides power to move a drive plunger motor 40 under control of digital commands from the CPU 10. The servo amplifier 24 drives a plunger motor 40 which is driven by filtered direct current derived from a series of pulse width modulated current pulses. To drive the plunger motor 40, a D.C. voltage source 42 supplies current to the motor 40 by feeding the respective collectors of transistors 46 and 56. To drive the plunger motor 40 in a forward direction, a position error detect circuit 50 switches on transistor 46 and a pulse-width modulating control circuit 52 delivers a series of enabling pulses to the field effect transistor (FET) 48.

The error detect circuit 50 generates follows a position error signal from the servo control 22 via a conductor 54. During forward driving of the plunger motor 40, a corresponding set of transistors 56 and 58 is switched off. This allows d.c. current pulses to flow in a forward direction through the plunger motor 40.

To drive the plunger motor 40 in a reverse direction, the error detect circuit 50 switches on transistor 56 and the pulse width modulating circuit 52 pulses the field effect transistor 58. In this fashion, the plunger motor 40 is driven in the reverse dierction by the supply of d.c. current pulses in an opposite direction therethrough. Instead of employing FETs 48 and 58, a set of silicon controlled rectilius also can be used.

The position error signal supplied to the servo amplifier 24 over the conductor 54 indicates whether the plunger is ahead of or behind the desired position, and also controls the drive transistors 46, 56, 48 and 58 in an appropriate fashion to that the plunger motor 40 causes the plunger (not shown) of the syringe to track the desired position to maintain the appropriate flow rate, pressure and/or duration. The position error signal is proportional to the magnitude of the difference between the actual position of the plunger and the desired position of the plunger. As the difference increases, so does the width of the drive pulses supplied by the pulse-width modulating circuit 52 to the drive transistors 58 and 48. Thus, when the position error signal is large, the average current supplied to the plunger motor also increases.

The servo amplifier 24 also includes a pressure limit circuit 60 which functions to inhibit the pulse-width modulating circuit 52 and shut down the drive current pulses supplied to the plunger motor 40. The circuit 60 constitutes a control circuit which informs the servo control when the pressure in the syringe exceeds a given set point established by the processor 10. It does so by monitoring motor current which is proportional to the fluid pressure, and operates to reduce the motor velocity of the plunger motor 40 by cutting the duty cycle of pulse-width modulated current pulses when the pressure exceeds a preset limit. The injector device has pressure input means to set and/or display the pressure limit in conventional units of PSI, KPA, KG or ATU. A switch is also provided to select and/or display the selected unit.

Under normal conditions, when the pressure limit has not been exceeded, the servo amplifier 24 causes the plunger position to follow the position command established by the processor 10. A digital-to-analog converter device 74 in the servo control portion of the system sets the pressure limit command. When actual pressure exceeds the programmed pressure limit, the pressure limit circuit 60 asserts a pressure limit indication signal 64 which informs the CPU 10 to stop changing the position command signal. The pressure limit command signal is supplied to the servo amplifier 24 via conductor 62. When the pressure limit signal 64 is no longer present, the CPU 10 retakes control by resuming the transmission of position command signals starting from the known plunger position. The plunger position, derived from a rotary potentiometer or optical encoder, is monitored by a plunger position circuit 66, also embodied in the servo amplifier 24. The output of the plunger position circuit 66 is supplied to the servo control 22 via a conductor 68.

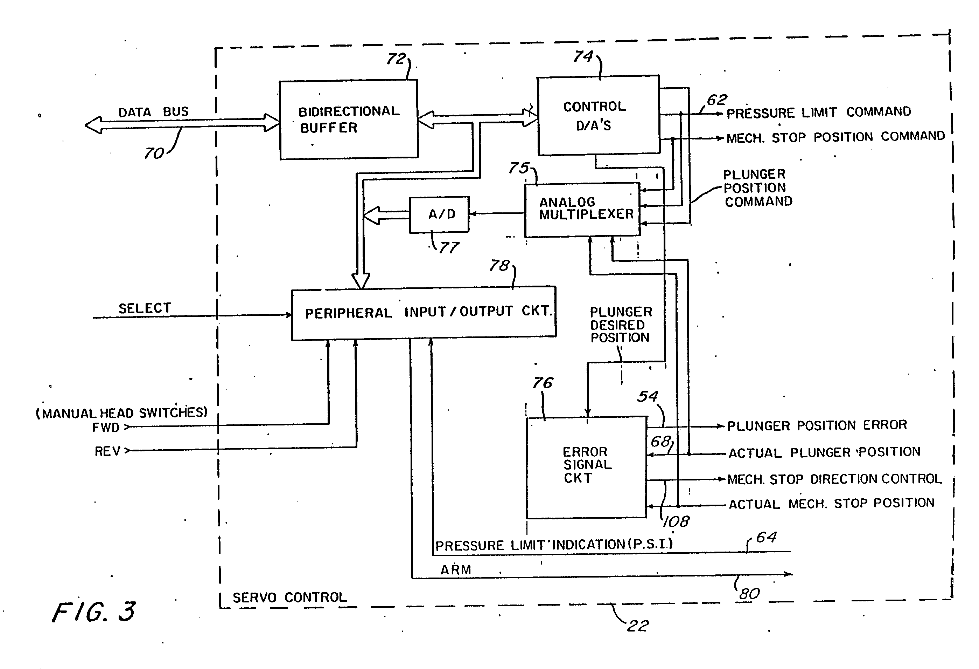

Figure 3 depicts a servo control circuit 22 in greater detail. The servo control circuit 22 provides an interface between the digital and analog components of the angiographic device. The CPU 10 provides digital position command signals via conductor 70 to a driver bidirectional buffer 72. The digital position command signals are eventually converted to an analog signal by a digital-to-analog converter 74 which are compared by a comparison network in the error circuit 76 which then appears as position error signal on conductor 54. As previously indicated, the plunger position error signal on conductor 54 is supplied to the servo amplifier 24 of Fig. 2.

To perform control and monitoring functions, an analog multiplexer 75, under control of the CPU 10 or a clocking signal, selectively conveys analog output signals from the control D/A circuit 74 or error circuit 76 to an A/D converter 77. The A/D converter 77 converts these signals to digital form and then, in turn, passes it to the CPU 10. The CPU 10 uses these digital signals to perform such functions as calibration, self-testing, and servo control. These functions are subsequently described.

The servo control circuit 22 also regulates the operation of the interlocked mechanical stop/plunger relay in the servo amplifier circuit 24 and the mechanical stop controller circuit 26. A peripheral input/output device 78 in the servo control circuit 22 provides means for transferring status and control signals to alter the operation of the servo amplifier circuit 24 and the mechanical stop controller 26. With respect to the interlocked mechanical stop/plunger relay operation, the input/output circuit 78 removes the plunger ARM signal via conductor 80 when the plunger reaches a predetermined position as determined by the CPU 10 and monitored by the plunger position circuit 66 (Figure 2). An ARM signal supplied over the conductor 80 is conveyed to the mechanical stop controller circuit 26 to actuate a mechanical stop drive motor which inhibits further plunger movement. This feature also will be subsequently described. The servo control circuit 22 also generates pressure command signals and monitors the pressure limit.

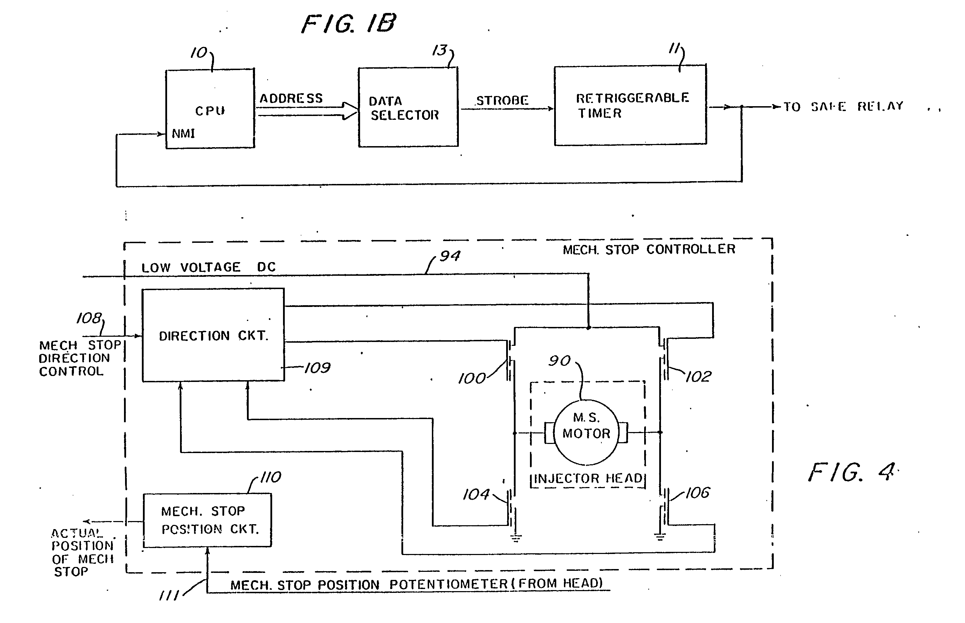

Figure 4 depicts the mechanical stop controller 26 wherein a mechanical stop motor 90 couples a mechanical stop member to actuate it against the plunger drive mechanism to block movements thereof when a predetermined calculated volume of contrast media has been injected.

Similarly to the plunger drive motor 40 of Figure 2, in a preferred embodiment, the mechanical stop motor 90 turns a ball screw to drive a mechanical stop plate forward. The drive command is given to drive the mechanical stop motor 90 at full speed until the mechanical stop position indicator on the mechanical stop mechanism indicates that desired position has been attained. This is accomplished by converting a potentiometer position signal supplied over conductor 111 from the stop mechanism to a digital value via an analog/digital converter. When the position signal indicates engagement of the stop plate, the drive command is removed.

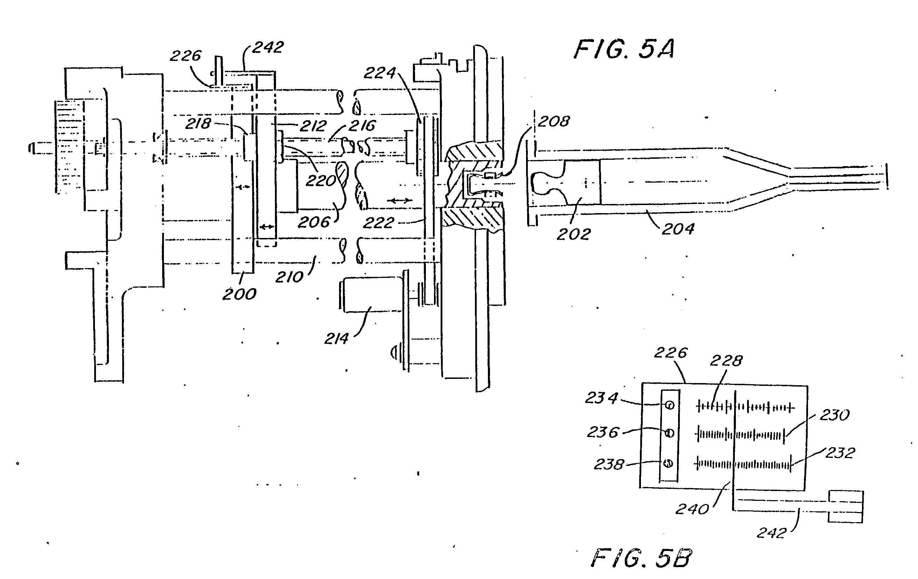

Figure 5A depicts an illustrative mechanical assembly for driving the piston of a syringe containing contrast media. As seen, a movable drive plate 200 actuates a piston 202 of a syringe 204 by way of a shaft 206. Although shown in spaced-apart relation, the piston 202 connects to a spring clip 208 when engaged therewith. The spring clip 208 is fastened to the end of the shaft 206. A plunger drive motor (not shown) corresponding to the motor 40 (Fig. 4) of the controller actuates the shaft 206 through the plunger plate 200. Linear movement of the plunger plate is guided by a guide rod 210.

As previously indicated, a mechanical stop prevents further movement of the drive shaft when a fixed amount of contrast media has been injected. To accomplish this task, a stop member in the form of a plate 212, is prepositioned prior to an injection. Prepositioning is done by a stop motor 214 (corresponding to motor 90 of Fig. 4) under control of the CPU 10. Conventional position transducers in the form of resistive potentiometers supply position information of the stop member 212 to the CPU 10 through an A/D converter.

Actuation of the stop member 212 is done by rotation of shaft 216 having threads mechanically coupled to a bore that is journalled through a bushing 218 held against the stop plate 212 by a nut 220. To rotate the shaft 216, motor 214, when energized, turns a grooved, flexible drive belt 222, which in turn, rotates a drive pulley 224. The pulley 224 connects to the shaft 216.

Figure 5B depicts a system for visually indicating the volume of contrast media injected from a syringe by utilizing different scales. Each scale corresponds to a syringe of a particular size, and the "active" scale is indicated by the appropriate light being energized. A face card 226 contains three separate scales 228, 230, and 232 of different units corresponding to incremental units of media in a syringe 204. Syringes of different diameters yield different units of media for the same linear movement of the plunger 202. To visually indicate which unit applies to a particular syringe, LED indicators 234, 236 and 238 are provided. One of these indicators is activated by a switch to show which scale is active, in which case, a needle 240 carried by arm 242 indicates relative volume injected. The arm 242 connects to the stop plate 212 while the face card 226 connects to the drive plate 200.

As previously indicated, the double-throw, double-pole operation of the relay 28 (Fig. 1A) energizes either the drive motor 90 (Fig. 4) or the plunger drive motor 40, but not both simultaneously. The mechanical stop motor 90 functions similarly to the plunger motor 40 of Figure 2, in that it includes a set of field effect transistors 100, 102, 104 and 106 which are energized to drive the mechanical stop motor 90 in a forward and/or reverse direction. Direction is controlled by a direction circuit 109 in response to a signal from the servo control 22 supplied via the conductor 108. The mechanical limit of the mechanical stop motor is controlled by a mechanical stop position circuit 110 which responds to a limit switch when the stop plate reaches a forward-most position or rearward-most position.

The CPU 10 controls the system using a microprocessor and associated program for monitoring or eliciting input information (e.g. injection parameters) supplied by an operator and then develops command information to control the injector device. The CPU 10 communicates with the I/O module 12 which interfaces external switches on the front control panel of the system.

The watchdog circuit 30 implements an active system for inhibiting injection of contrast media in the event of a microprocessor failure. The circuit 30 includes a re-settable timer which opens the safety relay 44 after a predetermined time period unless the CPU 10 actively resets it, thus allowing this cycle to repeat. Thus, if the CPU 10 fails, the safety relay 44 will be deactivated without further operation of the CPU 10.

The I/O module 12 monitors a manual signal supplied by the operator via a manual start switch 12 or an event signal from an external device supplied over the conductor 114. As previously indicated, the automated angiographic injector device may respond to certain cardiac events by monitoring an ECG signal thereby to actuate, or start an injection, at a predetermined time instance during an ECG cycle. This is done using well known techniques by the external event signal, and is particularly useful during certain cardiac studies.

The control/display panel 14 provides user/device interface for inputting and displaying injection parameters, such as syringe size, flow rate, volume, duration, pressure limit, or rise/fall time. This information can be prompted by the CPU 10 and entered by the operator. The entries are echoed back to the operator for a visual verification on a display of the panel 14. In physical construction, the panel 14 comprises a sealed membrane switch panel that is impervious to fluid spills and wipes clean without damage to switches contained underneath. Prior to an injection, the injection parameters are displayed and verified by an operator. Verification enables, e.g. arms, the injector device, and is preferably accomplished manually by depressing a contact switch associated with each injection parameter. After an injection, the actual values of flow, volume, etc. can be displayed so that the operator may confirm an effective or safe injection.

A pre-programmed injection module 18 provides non-volatile storage of injection parameters. Specific routine angiographic procedures require established flow rates, volumes, pressures and linear rise/fall time, but once these parameters are established, set-up of the angiographic device becomes routine. As an added convenience, these parameters can be stored in the pre-programmed injection module 18 so that an injection can proceed immediately after arming the system without the necessity to re-enter the injection parameters. To enhance reliability of the pre-programmed injection module 18, the stored injection parameters are verified by duplicating the values thereof in an auxiliary memory. Thus, the pre-programmed injection module preferably comprises a primary and a secondary random access memory for storing duplicates of the injection parameters and, prior to an injection procedure, the CPU 10 compares the contents of both memories to verify the parameters. Both memories are maintained with batteries when power is shut down thereby achieving non-volatility.

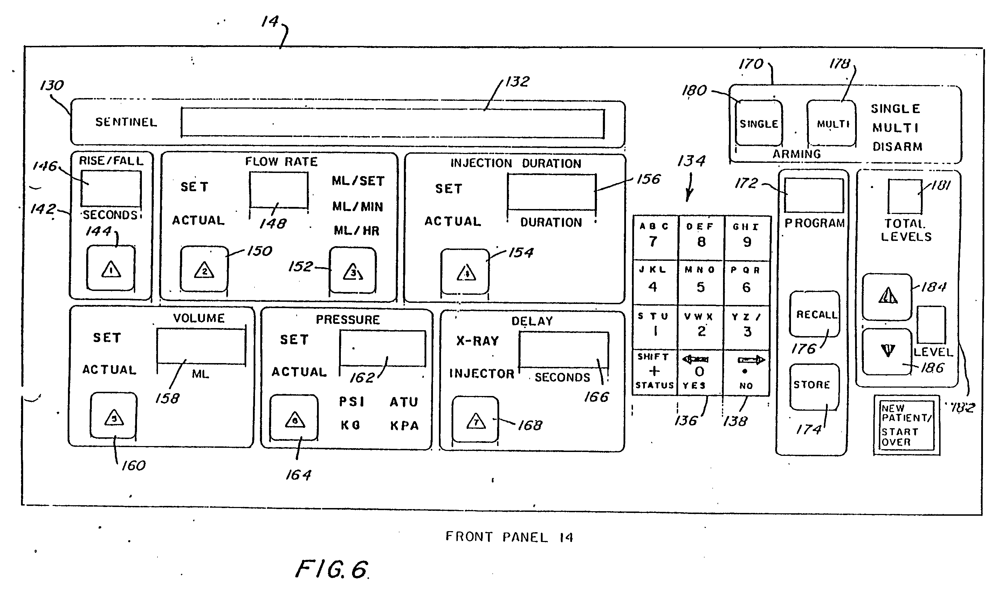

Figure 6 depicts a preferred arrangement of the front panel 14 of Figure 1 for providing an indication of injection parameters entered, confirmed and/or displayed in the device, and for providing an indication of control/injection parameters under which an injection had taken place. The front panel 14 preferably comprises a flat, sealed membrane covering switches located beneath the flexible membrane. This structure seals the switches from possible contamination from an external environment.

The front panel 14 includes various sections to facilitate entry of injection parameters and for displaying the status of an injector procedure. Display and entry of information is preferably accomplished in human readable format. Specifically, the front panel 14 includes a sentinel 130 which includes an alphanumeric display section 132. The display section 132 displays, among others, alphabetical and numeric information which is entered into the injector device by way of an alphanumeric keypad section 134. The keypad 134 enables entry of both numeric and character information in a conventional manner. During entry, the information is displayed on the sentinel display section 132.

The front panel also includes a dual function control and display section including an individual input pad 142 for entering and/or confirming, among other parameters, a rise/fall time of flow of contrast media. In operation, once the switch pad 144 is depressed and the desired rise/fall injection parameter is entered into the device, the specific rise/fall injection parameter is displayed in the display section 146. Similarly, a desired flow rate also can be programmed into the injection device. This injection parameter is displayed in a flow rate display 148 and entered into the device by switch input pad 150. The units of flow rate can be entered or displayed in milliliters per second milliliters per minute, or milliliters per hour under control of a switch pad 152.

The duration of an injection can be entered by depressing the switch pad 154 and then pressing "number" keys on keypad 134. Injection duration is displayed in a display portion 156. Any two of the three parameters, flow, volume, duration, may be entered in any order, and the third then automatically is calculated.

The volume of contrast media to be injected during an injection sequence is displayed in display 158 and is entered and/or confirmed by the switch pad 160. Similarly, the pressure at which the contrast media is to be injected is displayed and/or entered, respectively, by display 162 and switch pad 164. Similarly, x-ray photo delay from the time an injection sequence begins is displayed on display 166 and is entered with switch pad 168 and numerical keypad 134.

To facilitate entry of the injection parameters, the CPU 10 effects flashing of indicator lights located above the switch pads 144, 150, 152, 154, 160, 164 and 168. For example, when the CPU 10 prompts the operator to enter the rise/fall injection parameter, an indicator light above the switch pad 144 flashes. When the appropriate value has been entered and is displayed correctly in the display 146, the switch pad 150 is depressed by the operator, whereupon display 146 ceases to flash and stays lit, while the indicator light over the switch pad 150 begins flashing. The flashing sequence advances to the next injection parameter to be entered as each parameter is entered.

If a processor-recognizable parameter is not entered, the CPU 10 effects generation of an alarm, such as an audible beep and/or error message on display 132. Further, the CPU 10 will prevent further entry or arming of the device until the error has been corrected. As an example, entry of an injection volume that is greater than the syringe size will cause an error. The panel 14 also has a contact switch for clearing the contents of any parameter or information displayed or entered into the injector device.

The front panel 14 further includes an arming section 170 for selecting a single phase or a multiphase injection sequence. In order to recall routine injection parameters or to store a set of injection parameters, a program section 172 is provided. In operation, once a set of injection parameters is loaded into the device and displayed in the various parameter sections, an identification tag can be generated by way of the keypad 134, displayed in the sentinel 132, and stored in non-volatile memory by depressing the store switch 174. One or more numbers or words, or alphanumeric combinations, can be used to identify a prestored set of injection parameters. The set of injection parameters then is stored in a storage module under the name or number which appears in the sentinel 132 when stored. That same information can be quickly recalled by entering the name or number associated with the previously stored set of injection parameters by depressing recall switch 176.

Then, before the injector can be armed for performing an injection, the operator is required to depress each of the switch pads 144, 150, 152, 154, 160, 164 and 168, to verify the accuracy of the injection parameters being displayed.

During a multi-phasic injection sequence, a number of injections may be performed without disarming the machine after delivery of the programmed injection volume. Each depression of the main start switch 112 will deliver the same volume until insufficient volume remains in the syringe. A switch pad 178 in the arming section activates the device for a multi-phasic injection sequence. A level control section 182 indicates the number of levels in a single phase or multiphasic injection sequence. The specific level may be incremented or decremented by way of switch pads 184 and 186 and parameters for each level are entered on switch pads 144, 150, 152, 154, 160, 164, and 168. During a single phase injection, the rise/fall time, flow rate, volume, and pressure for each level are delivered until all levels (up to 9) are completed or the injection is terminated. During multi-phasic injections, all levels are delivered in continuous sequence unit the injection is complete.

Each time the start switch 112 is pressed, the same injection is delivered without first rearming the machine via multi-arm keypad 178.

The central processing unit 10 also provides a self-calibration and a self test feature. Self-calibration allows proper servicing of critical analog/digital and digital/analog converters in the system. Upon calling up of appropriate software routines, the processor commands a digital/analog converter 74 to transmit a corresponding analog value, which is then switched through an appropriate analog/digital converter 74. The converted value is then compared with the original digital value thereby to allow adjustment of gain and offsets in the servo control system.

The injector allows the user to enter a syringe size which is used to automatically adjust internal machine parameters to deliver the programmed flows, volumes, and pressures. Syringe size entry is made by answering questions posed by the sentinel display 132 although automatic detection of size may be accomplished by means of mechanical switches located behind the turret assembly.

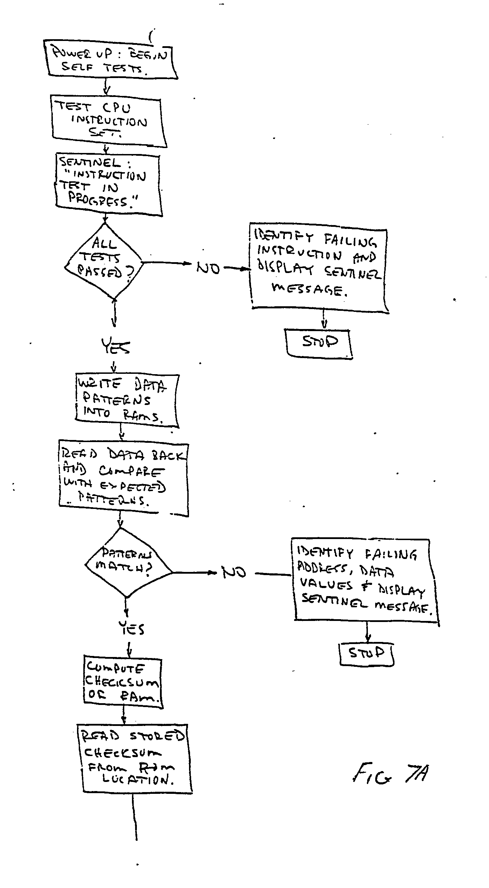

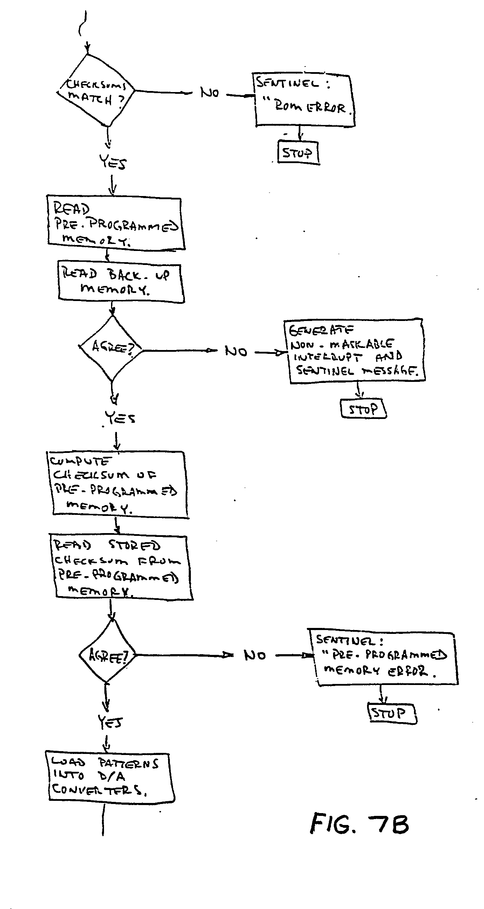

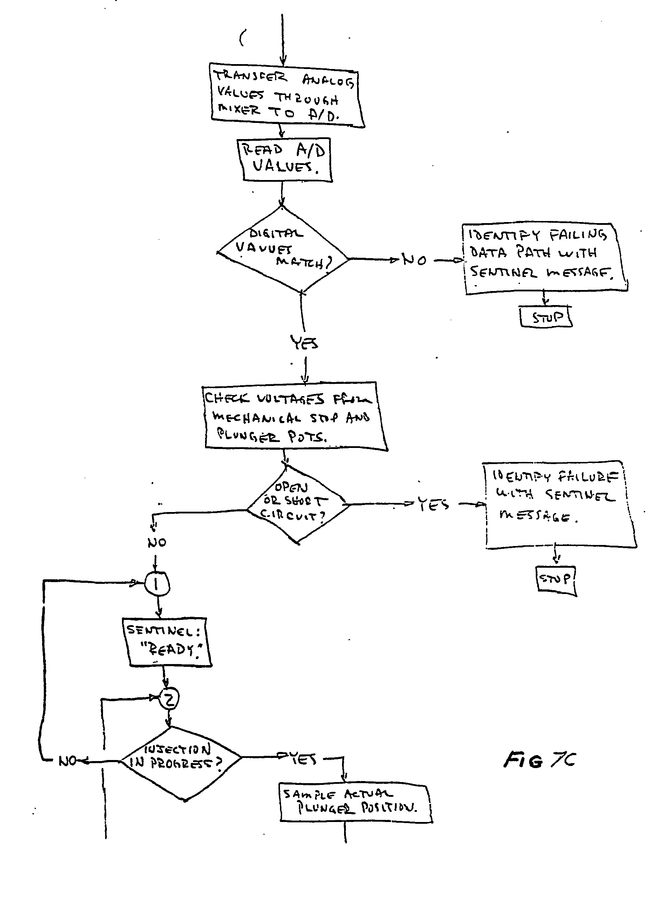

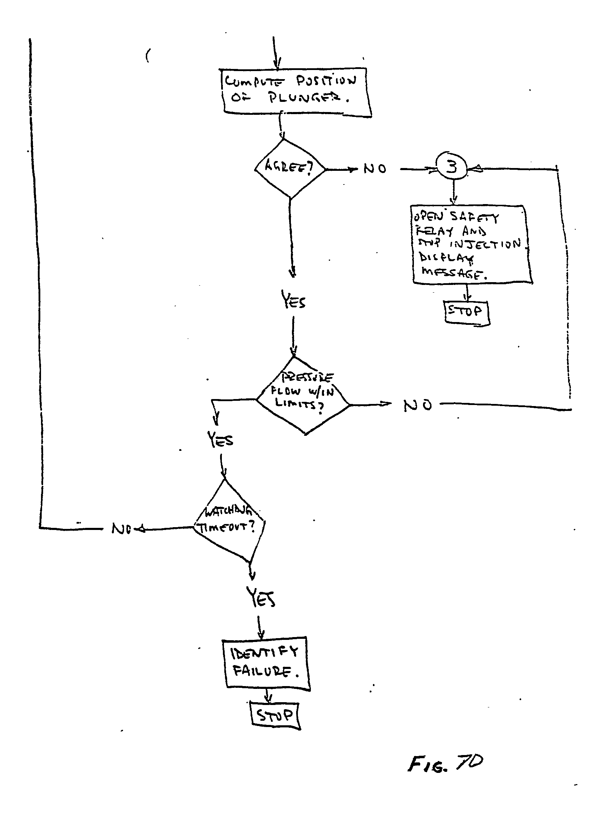

Likewise, a self-test feature of the invention embodies software routines executed by the central processor 10 which initiates checks of the electrical components, such as the memory, address and data busses, device decoding and checks the accuracy of the data conversion hardware. These functions are accomplished by conventional hardware and specialized software routines, the latter being described in the flow charts of Figures 7A, 7B, 7C and 7D. If failures are detected by CPU 10, an appropriate diagnostic message appears on sentinel display 132 to aid in repairing the machine.

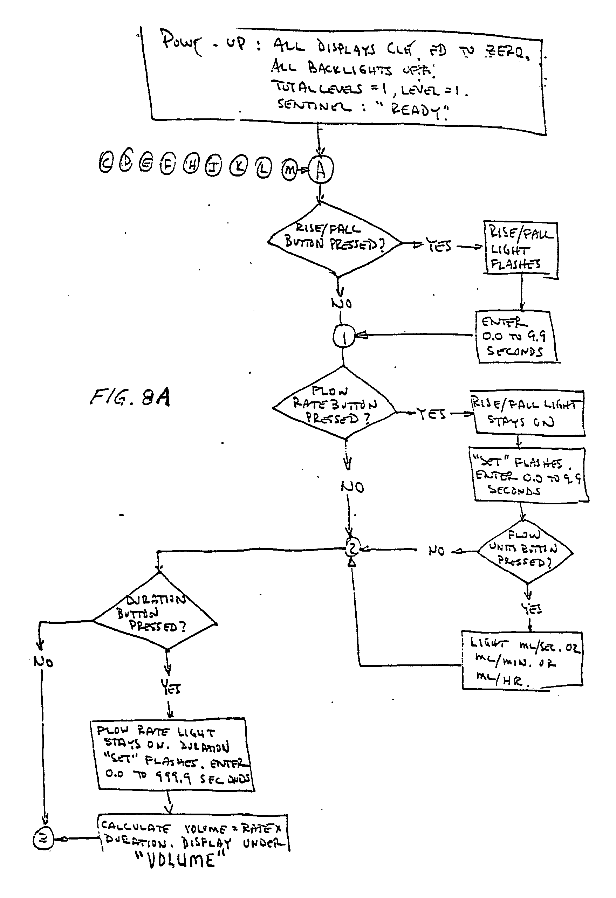

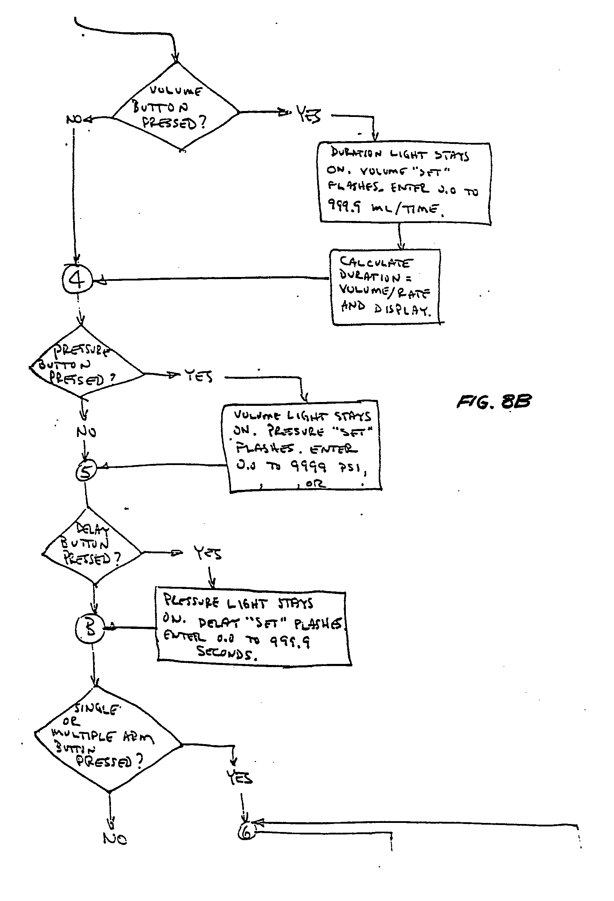

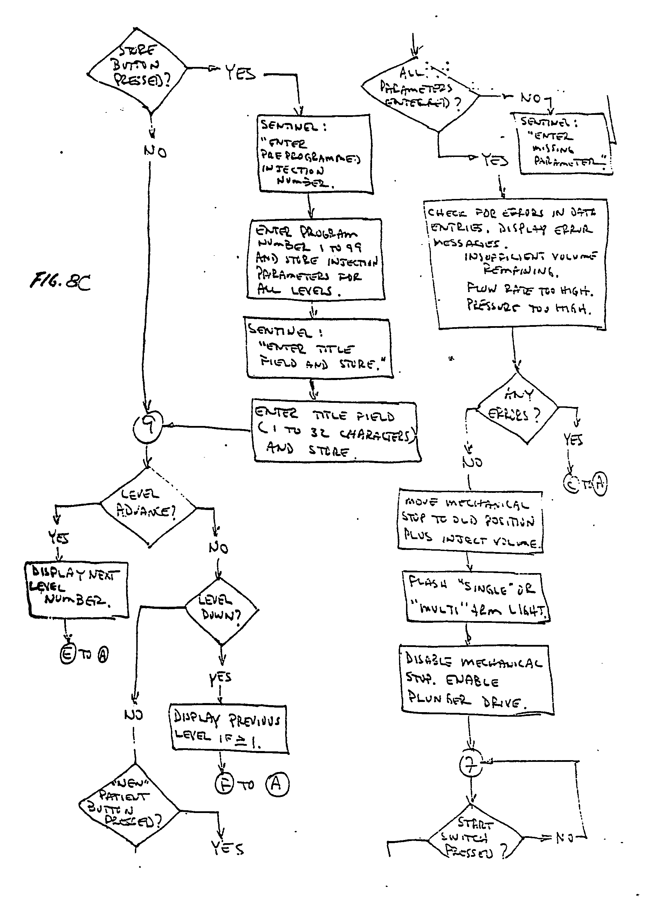

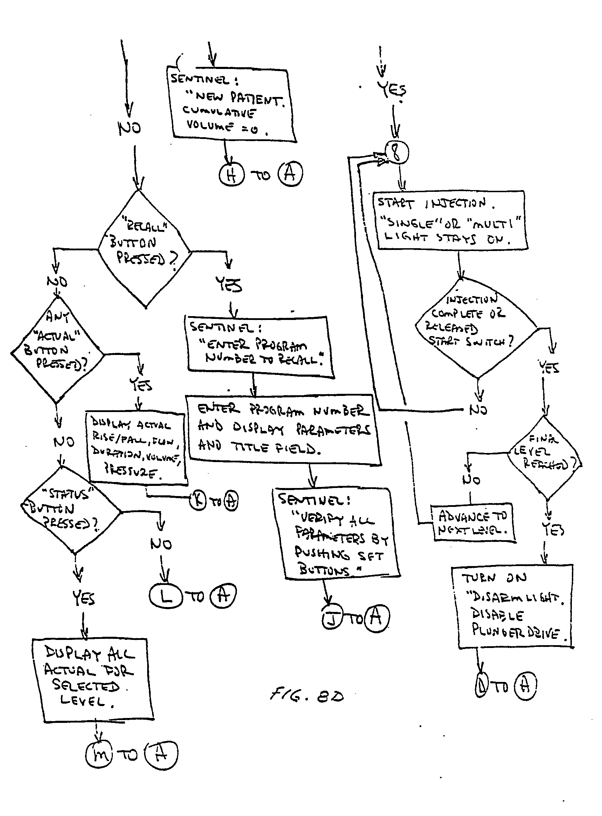

Figures 8A, 8B, 8C and 8D are flow diagrams showing the general operational sequence of the automated injector device for performing an injection as described above.

In view of the foregoing disclosure, it is seen that the specified advantages are obtained by providing automated control of an angiographic injector device. The disclosure hereof is illustrative and is not intended to limit the scope of the invention as defined by the appended claims. Several modifications, changes and adaptations can be made by those skilled in the art without departing from the scope of the invention as defined by the appended claims. For example, the data acquisition modules essentially comprises analog-to-digital and digital-to-analog converters, but are not limited to the same. These may be substituted by other types of data acquisition units to obtain information to provide the control of duration, flow rate and pressure of contrast media. Likewise, a closed-loop servo control system is shown, but the invention may be practiced with other types of control systems. Accordingly, it is the intent of each inventor to include all such modifications as may come within the true scope of the invention which is defined by the appended claims.

**Claims**

1. An angiographic injector device for injecting contrast media into the vascular system of a patient, said device comprising:

means (204) for holding a reservoir containing contrast media and including a discharge port through which the media is discharged, and

drive means (24) for effecting discharge of said media through said discharge port, characterized in that it further comprises:

receiving means for receiving at least one injection control parameter which includes at least one parameter selected from a group including flow rate of contrast media, volume of contrast media, duration of an injection, pressure limit of said contrast media, x-ray photo delay, inject delay and flow rise/fall time of pressure of contrast media,

processor control means (10) for generating at least one injection control signal as a function of said at least one injection parameter supplied thereto and for supplying said control signal to said drive means (24) thereby to effect controlled discharge of said media,

wherein said drive means (24) and processor control means (10) comprise a closed loop servo system responsive to a position command signal produced by said processor control means (10).

2. An angiographic injector device as recited in claim 1, wherein said injection control parameter is the pressure of the media in the reservoir and wherein said drive-means (24) and processor control means (10) comprise pressure limit means (60) responsive to a pre-established pressure limit signal for inhibiting the responsiveness of said servo system to said position command signal when the pressure in said reservoir exceeds said pre-established limit.

3. An angiographic injector device as recited in claim 2, wherein said pressure limit means includes means to receive said pre-established pressure limit signal in units of PSI, KPA, KG, or ATU and said angiographic injector device further includes switch means for selecting one of said units indicative of said pre-established pressure limit signal.

4. An angiographic injector device as recited in claim 1 or 2, wherein said reservoir comprises a syringe having a piston (202) for forcing media through the discharge port thereof, said drive means comprises a d.c. servo motor (40) operatively connected to said piston, and said control means includes means for supplying to said d.c. motor a d.c. current (52) derived from a pulse-width-modulated drive current having duty cycle that is proportional to the difference between the actual and the desired positions of said piston during an injection.

5. An angiographic injector device as recited in claim 1 or 4, further including mechanical stop controller means (26) responsive to a pre-established forward limit of said piston to inhibit said drive means for discharging media from said syringe, said forward limit being determined by said processor control means.

6. An angiographic injector device as recited in claim 5, wherein said mechanical stop controller means comprises a stop member (212) and a d.c. stop motor (214) operatively connected to said stop member, means (66) for monitoring the position of said piston, means (74) for producing a stop command signal when the position of said piston reaches said forward limit, and means responsive to said stop command signal to actuate said stop motor which, in turn, advances said stop member to mechanically prevent forward movement of said piston.

7. An angiographic injector device as recited in claim 6, wherein said processor control means includes control means (110) for establishing said pre-established forward limit of said stop member in dependence on the value of an injection parameter supplied thereto.

8. An angiographic injector device as recited in claim 7, further including safety relay means (28, 44) for alternatively enabling one of said drive means and said mechanical stop controller means thereby to prevent movement of said drive means while said mechanical stop controller is actuated.

9. An angiographic injector device as recited in claim 8, further comprising an indicator light for visually indicating the position of said mechanical stop member and a second indicator light corresponding to the position of said piston relative to said first indicator light.

10. An angiographic injector device as recited in claim 1, further comprising:

watchdog means (30) for inhibiting said drive means in response to a failure of said processor control means.

11. An angiographic injector device as recited in claim 10, wherein said watchdog means comprises resettable timer means responsive to a reset signal for producing a shut-down signal after a predetermined time interval, and said processor control means includes means for periodically producing said reset signal whereby to inhibit passively said drive means in the event of failure of said processor control means.

12. An angiographic injector device as recited in claim 1 or 11, wherein said processor control means includes means for eliciting at least one injection parameter from an operator of said injector device thereby to facilitate use thereof.

13. An angiographic injector device as recited in claim 12, wherein said at least one injection parameter includes at lesat one parameter selected from a group including flow rate of contrast media, volume of contrast media, duration of an injection, pressure limit of said contrast media, x-ray photo delay, inject delay and flow rise/fall time of pressure of contrast media.

14. An angiographic device as recited in claim 1 or 13, including receiving means having a sealed, flat membrane control panel (14) with individual input pads associated with each of said at least one injection parameter, each said input pad being operable for inputting into said control means an associated injection parameter.

15. An angiographic device as recited in claim 14, wherein said processor control means further includes arming means (170) responsive to the actuation of each of said at least one input pad for enabling said drive means and for inhibiting said drive means when any one of said at least one said input pad is not actuated.

16. An angiographic injector device as recited in claim 1 or 13, further comprising: primary memory means (18) for storing said at least one injection parameter, secondary memory means for storing a duplicate of the contents of said at least one injection parameter, and parameter verification means for comparing the injection parameters of said primary and secondary means and for inhibiting said drive means when a mismatch in stored injection parameters occurs.

17. An angiographic injector device as recited in claim 1 or 13, further comprising memory means (18) for storing a set of injection parameters, means for producing an identification tag for each set of said at least one injection parameters and for storing said tag with said set in said memory means, recall means responsive to a given tag for recalling from said memory means an associated set of injection parameters and for supplying said recalled set of injection parameters to said receiving means thereby to enable quick recall of routine sets of injection parameters without operator input prior to an injection.

18. An angiographic injector device as recited in claim 17, further including means for recalling and sequencing through each of said recalled injection parameters of a given set and for eliciting information to amend a particular injection parameter.

19. An angiographic injector device as recited in claim 17, further including means for verifying each of said recalled input parameters prior to an injection.

20. An angiographic injector device as recited in claim 19, wherein said means for verifying comprises a manual switch associated with each of said at least one injection parameter.

21. An angiographic injector device as recited in claim 19, wherein said means for verifying comprises redundant memories for storing each of said injection parameters, means for comparing the contents of said injection parameter stored in said redundant memories, and means for disabling said injector device or alerting the operator in the event of a difference in said comparison of data between said redundant memories.

22. An angiographic injector device as recited in claim 21, wherein each of said redundant memories comprises a random access memory powered by a separate battery.

23. An angiographic injector device as recited in claim 16, wherein said primary and secondary memory means includes means for retaining said injection parameters in the absence of power to said injector device.

24. An angiographic injector device as recited in claim 1, further comprising: interface means (20) connected to said processor control means for transferring status information or an injection parameter with said injector device.

25. An angiographic injector device as recited in claim 24, wherein said interface means includes a universal asynchronous receive/transmit port for providing communication with an external device.

26. An angiographic injector device as recited in claim 1, wherein said control means includes multi-level injection control means for sequentially changing a given set of injection parameters during an injection.

27. An angiographic injector device as recited in claim 26, further including means (142) for pre-establishing a rise/fall time of pressure level of said contrast media between successive phase of a multi-phase injection sequence.

28. An angiographic injector device as recited in claim 1, further comprising: self-calibration means including means for producing a test command signal to position said drive means at a test position, said monitoring means being operative to detect the actual position of the drive means as the operational condition, means for comparing the test position with the actual position, and means for re-calibrating said drive means on the basis of said comparison.

29. An angiographic injector device as recited in claim 1, further including: self-test means for automatically checking the accuracy of said control means and for producing an indication of a predetermined deviation from a desired accuracy and for inhibiting an injection in response thereto.

30. An angiographic injector device as recited in claim 29, wherein said self-test means comprises means for checking the operational condition of said processor control means and said memory means.

31. An angiographic injector device as recited in claim 30, wherein said self-test means comprises means for executing instructions of said processor control means using test data and means for checking the results of each said executed instruction with a pre-established correct result.

32. An angiographic injector device as recited in claim 30, wherein said self-test means comprises means for writing known data in said memory means, means for reading back said known data written to said memory means, and means for comparing the read back data and the known data thereby to check the operational status of said memory means.

33. An angiographic injector device as recited in claim 30, wherein said memory means comprises a read only memory and said self-test comprises check means for verifying the contents of said read only memory.

34. An angiographic injector device as recited in claim 30, wherein said self-test means further comprises analog-to-digital and digital-to-analog testing means for checking the accuracy of sensors, A/D converters and D/A converters of said injector device.

35. An angiographic injector device as recited in claim 30, wherein said self-test means sends a digital word to be converted to an analog signal to a digital-to-analog converter, and means for verifying the accuracy of said digital-to-analog converter on the basis of said transmitted information.

36. An angiographic injector device as recited in claim 34, wherein said self-test means generates and transmits a calibrated analog signal to an A/D converter and means for verifying the accuracy of the digital signal converted by said A/D converter.

37. An angiographic injector device as recited in claim 34, wherein said self-test means further comprises means for checking said pressure limit means.

38. An angiographic injector device as recited in claim 30, wherein said self-test means further comprises means for monitoring current supplied to said drive means on the basis of a calibrated controlled command current setting thereby to verify the operation of said drive means.

39. An angiographic injection device as recited in claim 1, wherein said monitored operation condition is an ECG waveform representative of a cardiac cycle of the patient, and means for automtically effecting injection of a given quantity of media at a controlled rate during a given time interval of said cardiac cycle thereby to facilitate x-ray photography that is synchronized with said ECG waveform.

40. An angiographic injection device as recited in claim 1, including a flat, sealed membrane control panel (14) having at least one input pad for each of said at least one injection parameter, said input pads being operable for entering said injection parameter.

41. An angiographic injector device as recited in claim 1, comprising a sealed, flat membrane control panel (14) having a human readable format section to dispaly operator inputs and to indicate output information pertaining to the injector device, a dual function control and display panel section for entering and displaying injection parameters, a numerical keypad section (134) for entering alpha numeric information, a pre-programmed injection section for storing pre-established injection parameters, and an arming control sechion (170) for enabling said drive means in response to information stored in said injection device and operator input.

42. An angiographic injector device as recited in claim 41, wherein said dual function control and display panel section includes means for clearing said display panel section and said human readable format section of information.

43. An angiographic injector device as recited in claim 41, further comprising a multi-phasic injection control section (182) for receiving at least two sets of injection control parameters, and means for successively effecting injection in accordance with each set of injection control parameters.

44. An angiographic injector device as recited in claim 41, wherein said control means comprises means for eliciting from an operator a number of injection parameters, and said dual function control and display panel section comprises an indicator light associated with each said injection parameter, means operable during elicitation of each said input parameter for effecting flashing of said indicator light for alerting the operator of a particular injection parameter to be entered, and means for terminating said flashing when the injection parameter has been entered and for effecting flashing of a next successive indicator light associated with a next successive input parameter is to be entered by the operator.

45. An angiographic injector device as recited in claim 44, wherein said control means further comprises means (76) for recognizing errors of an injection parameter entered by an operator, and means for generating an audible alert signal upon recognition of said error.

46. An angiographic injector device as recited in claim 1, wherein said reservoir comprises a syringe having a plurality of scales (228, 230, 232) disposed on a side wall thereof and an indicator light (226) for indicating a selected scale whereby to enable visual observation of volume injected during an injection procedure.

47. An angiographic injector device as recited in claim 1, wherein said control means includes means (182) for effecting multi-phasic injection sequences wherein, during each injection phase, the injection of contrast media is controlled according to individual sets of injection parameters successively supplied to said control means.

48. An angiographic injector device as recited in claim 47, wherein between each phase of said multi-phasic injection, the rise/fall magnitude of pressure of contrast media is controlled at a predetermined level.

49. An angiographic injector device as recited in claim 48, further including means for verifying each parameter of a set of injection parameters between successive phases of a multi-phasic injection sequence.

50. An angiographic injector device as recited in claim 1, further comprising:

control means for regulating the discharge of said contrast media, said control means further including self test means for monitoring an operational state of said angiographic injector device and for producing an error signal in the event that a fault is detected.

51. An angiographic injector device as recited in claim 50 including memory means (18) for storing information pertaining to said injection and A/D and D/A convertors for interfacing analog and digital control and status signals, wherein said monitored condition includes the operational state of said processor control means, said memory, and said A/D and D/A convertors.

52. An angiographic injector device as recited in claim 50, wherein said monitored condition includes excessive pressure of said contrast media during an injection, excessive current drawn by said drive means, excessive positional error of said drive means, and said control means is responsive to inhibit said injector device in the event that said operational states are excessive.

53. An angiographic injector device as recited in claim 1, wherein said control means further includes computer interface means (20) for providing external communication with said angiographic injector device.

54. An angiographic injector device as recited in claim 53, wherein said computer interface means comprises a data channel for allowing external communication with said external device for transferring information including control and status information.

55. An angiographic injector device as recited in claim 54, wherein said external computer interface comprises a universal asynchronous receiver/transmit port.

**Patentansprüche**

1. Angiographischer Injektor zum Injizieren von Kontrastmittel in das Gefäßsystem eines Patienten, welcher Injektor umfaßt:

eine Einrichtung (204) mit einem Vorratsbehälter für Kontrastmittel und einer Auslaßöffnung, durch die hindurch das Mittel abgegeben wird, und

einen Antrieb (24) zur Abgabe des genannten Mittels durch die genannte Auslaßöffnung dadurch gekennzeichnet, daß der Injektor außerdem umfaßt:

eine Empfangseinrichtung zum Aufnehmen von mindestens einem Injektions-Steuerparameter, der mindestens einen Parameter aus der Gruppe der folgenden Parameter umfaßt: Durchflußmenge des Kontrastmittels, Volumen des Kontrastmittels, Dauer einer Injektion, Druckbegrenzung des genannten Kontrastmittels, Verzögerung der Röntgenaufnahme, Verzögerung der Injektion und Anstiegs-/Abfallzeit des Strömungsdrucks des Kontrastmittels,

eine Steuereinheit (10) zum Erzeugen von mindestens einem Injektions-Steuersignal in Abhängigkeit von dem genannten mindestens einen zugeführten Injektionsparameter und zum Weiterleiten des genannten Steuersignals zu dem genannten Antrieb (24), um auf diese Weise eine gesteuerte Abgabe des genannten Mittels herbeizuführen,

worin der genannte Antrieb (24) und die genannte Steuereinheit (10) einen geschlossenen Servo-Regelkreis bilden, der auf ein Stellungssteuersignal anspricht, das von der genannten Steuereinheit (10) erzeugt wird.

2. Angiographischer Injektor nach Anspruch 1, dadurch gekennzeichnet, daß der genannte Injektionssteuerparameter der Druck des Mittels in dem Behälter ist, und daß der genannte Antrieb (24) und die genannte Steuereinheit (10) eine Druckbegrenzungseinrichtung (60) umfassen, die auf ein Signal für einen voreingestellten Grenzdruck ansprechen, umsdas Ansprechen des genannten Servosystems auf das genannte Stellungssteuersignal zu verhindern, wenn der Druck in dem genannten Behälter den genannten voreingestellten Grenzwert übersteigt.

3. Angiographischer Injektor nach Anspruch 2, dadurch gekennzeichnet, daß die genannte Druckbegrenzungseinrichtung eine Einrichtung zum Aufnehmen des genannten Signals des voreingestellten Grenzdrucks in psi- kPa- kg- oder atu-Einheiten umfaßt, und daß der genannte angiographische Injektor außerdem eine Schalteinrichtung für die Wahl einer der genannten, das genannte voreingestellte Grenzdrucksignal anzeigenden Einheiten umfaßt.

4. Angiographischer Injektor nach Anspruch 1 oder 2, dadurch gekennzeichnet, daß der genannte Behälter eine Spritze aufweist, deren Kolben (202) das Mittel durch die Abgabeöffnung der Spritze drückt, daß der genannte Antrieb einen Gleichstromservomotor (40) aufweist, der in Wirkverbindung mit dem genannten Kolben steht, und daß die genannte Steuereinheit eine Einrichtung umfaßt, die dem genannten Gleichstrommotor einen Gleichstrom (52) zuführt, der von einem pulsbreitenmodulierten Treiberstrom abgeleitet ist, dessen Tastverhältnis proportional der Differenz zwischen der augenblicklichen und der gewünschten Stellung des genannten Kolbens während einer Injektion ist.

5. Angiographischer Injektor nach Anspruch 1 oder 4, weiter gekennzeichnet durch einen mechanischen Arretierregler (26), der auf eine voreingestellte vordere Grenze des genannten Kolbens anspricht, um den genannten Antrieb daran zu hindern, das Mittel aus der Spritze abzugeben, wobei die genannte vordere Grenze von der genannten Steuereinheit bestimmt wird.

6. Angiographischer Injektor nach Anspruch 5, dadurch gekennzeichnet, daß der genannte mechanische Arretierregler ein Arretierteil (212) und einen in Wirkverbindung mit dem genannten Arretierteil stehenden Gleichstrom-Arretiermotor (214) aufweist, eine Einrichtung (66) zum Überwachen der Stellung des genannten Kolbens, eine Einrichtung (74) zum Erzeugen eines Arretiersteuersignals, wenn die Stellung des genannten Kolbens die genannte vordere Grenze erreicht, und eine auf das genannte Arretiersteuersignal ansprechende Einrichtung, um den genannten Arretiermotor zu betätigen, der seinerseits das genannte Arretierteil vorschiebt, um die Vorwärtsbewegung des genannten Kolbens mechanisch zu verhindern.

7. Angiographischer Injektor nach Anspruch 6, dadurch gekennzeichnet, daß die genannte Steuereinheit eine Steuereinrichtung (110) umfaßt, die die genannte voreingestellte vordere Grenze des genannten Arretierteils in Abhängigkeit von dem Wert eines zugeführten Injektionsparameters einstellt.

8. Angiographischer Injektor nach Anspruch 7, weiter gekennzeichnet durch eine Sicherheitsrelaiseinrichtung (28, 44), die wechselweise den genannten Antrieb oder den genannten mechanischen Arretierregler betätigt, um dadurch die Bewegung des genannten Antriebs zu verhindern, während der genannte mechanische Arretierregler betätigt wird.

9. Angiographischer Injektor nach Anspruch 8, weiter gekennzeichnet durch eine Leuchtanzeige zum visuellen Anzeigen der Stellung des genannten mechanischen Arretierteils und eine zweite Leuchtanzeige, die der Stellung des genannten Kolbens gegenüber der genannten ersten Leuchtanzeige entspricht.

10. Angiographischer Injektor nach Anspruch 1, weiter umfassend:

eine Überwachungseinrichitung (30) zum Sperren des genannten Antriebs im Falle einer Störung der genannten Steuereinheit.

11. Angiographischer Injektor nach Anspruch 10, dadurch gekennzeichnet, daß die genannte Überwachungseinrichtung einen rückstellbaren Zeitgeber umfaßt, der auf ein Rückstellsignal anspricht zum Erzeugen eines Abschalt-Signals nach einem vorbestimmten Zeitintervall, und daß die genannte Steuereinheit eine Einrichtung zum periodischen Erzeugen des genannten Rückstellsignals umfaßt, wodurch der genannte Antrieb im Falle einer Störung der genannten Steuereinheit passiv gesperrt wird.

12. Angiographischer Injektor nach Anspruch 1 oder 11, dadurch gekennzeichnet, daß die genannte Steuereinheit eine Einrichtung aufweist, die den Operateur des genannten Injektors zur Eingabe mindestens eines Injektionsparameters veranlaßt, wodurch der Gebrauch des Injektors vereinfacht wird.

13. Angiographischer Injektor nach Anspruch 12, dadurch gekennzeichnet, daß der genannte mindestens eine Injektionsparameter mindestens einen Parameter umfaßt, der aus einer folgende Parameter umfassenden Gruppe gewählt ist: Durchflußmenge des Kontrastmittels, Volumen des Kontrastmittels, Dauer einer Injektion, Druckgrenze des genannten Kontrastmittels, Verzögerung der Röntgenaufnahme, Injektionsverzögerung und Strömungs-Anstiegs-/Abfallzeit des Drucks des Kontrastmittels.

14. Angiographischer Injektor nach Anspruch 1 oder 13, umfassend eine Aufnahmeeinrichtung mit einem angedichteten, ebenen Membransteuerfeld (14) mit einzelnen Eingabeblöcken, die jedem bzw. dem mindestens einen Injektionsparameter zugeordnet sind, wobei jeder der genannten Eingabeblöcke betätigt werden kann, um in die genannte Steuereinheit einen zugeordneten Injektionsparameter einzugeben.

15. Angiographischer Injektor nach Anspruch 14, dadurch gekennzeichnet, daß die genannte Steuereinheit außerdem eine Aktivierungseinrichtung (170) aufweist, die auf das Betätigen jedes bzw. des genannten mindestens einen Eingabeblocks zum Einschalten des genannten Antriebs und zum Sperren der genannten Antriebseinrichtung anspricht, wenn irgendein bzw. der genannte mindestens eine Eingabeblock nicht betätigt ist.

16. Angiographischer Injektor nach Anspruch 1 oder 13, weiter gekennzeichnet durch: einen ersten Speicher (18) zum Speichern des genannten mindestens einen Injektionsparameters, einen Zusatz-Speicher zum Speichern des Doppels des Inhalts des genannten mindestens einen Injektionsparameters, und eine Parameternachprüfungseinrichtung zum Vergleichen der Injektionsparameter des genannten ersten und des Zusatz-Speichers und zum Sperren des genannten Antriebs, wenn eine Fehlanpassung der gespeicherten Injektionsparameter auftritt.

17. Angiographischer Indikator nach Anspruch 1 oder 13, weiter gekennzeichnet durch einen Speicher (18) zum Speichern eines Satzes von Injektionsparameters, eine Einrichtung zum Erzeugen eines Identifikationsetiketts für jeden Satz der genannten mindestens einen Injektionsparameter und zum Speichern des genannten Etiketts mit dem genannten Satz in dem genannten Speicher, eine Rückholeinrichtung, die auf ein gegebenes Etikett anspricht, um von dem genannten Speichern einen zugehörigen Satz von Injektionsparametern zurückzuholen, und die den genannten zurückgeholten Satz von Injektionsparametern der genannten Aufnahmeeinrichtung zuführt, wodurch ein schneller Rückruf von Programmsätzen von Injektionsparametern ohne Eingabe durch den Operateur vor einer Injektion möglich wird.

18. Angiographischer Injektor nach Anspruch 17, weiter gekennzeichnet durch eine Einrichtung zum Rückholen eines gegebenen Satzes und Einreihen über jeden der genannten zurückgeholten Injektionsparameter zum Entnehmen von Information für die Berichtigung eines bestimmten Injektionsparameters.

19. Angiographischer Injektor nach Anspruch 12, weiter gekennzeichnet durch eine Einrichtung zum Überprüfen jedes der genannten zurückgeholten Eingabeparameters vor einer Injektion.

20. Angiographischer Injektor nach Anspruch 19, dadurch gekennzeichnet, daß die genannte Überprüfungseinrichtung einen Handschalter umfaßt, der jedem bzw. dem genannten mindestens einen Injektionsparameter zugeordnet ist.

21. Angiographischer Injektor nach Anspruch 19, dadurch gekennzeichnet, daß die genannte Überprüfungseinrichtung überzählige Speicher zum Speichern jedes der genannten Injektionsparameter umfaßt, ferner eine Einrichtung zum Vergleichen des Inhalts der in den genannten überzähligen Speichern gespeicherten Injektionsparameter und eine Einrichtung zum Abschalten des genannten Injektors oder zum Alarmieren des Operateurs, wenn ein Unterschied beim Datenvergleich zwischen dem genannten überzähligen Speichern auftritt.

22. Angiographischer Injektor nach Anspruch 21, dadurch gekennzeichnet, daß jeder überzählige Speicher einen von einer getrennten Batterie gespeisten RAM-Speicher aufweist.

23. Angiographischer Injektor nach Anspruch 16, dadurch gekennzeichnet, daß der genannte erste und der Zusatz-Speicher eine Einrichtung zum Sichern der genannten Injektionsparameter beim Stromausfall an dem genannten Injektor aufweist.

24. Angiographischer Injektor nach Anspruch 1, weiter gekennzeichnet durch: eine mit der genannten Steuereinheit verbundens Schnittstelle (20) zum Übertragen von Statusinformationen oder eines Injektionsparameters zu dem genannten Injektor.

25. Angiographischer Injektor nach Anspruch 24, dadurch gekennzeichnet, daß die genannte Schnittstelle einen Universal-Empfangs/Sende-Port zur Herbeiführung der Kommunikation mit einem Außengerät umfaßt.

26. Angiographischer Injektor nach Anspruch 1, dadurch gekennzeichnet, daß die genannte Steuereinheit eine Mehrphasen-Injektionssteuerung zum sequentiellen Ändern eines gegegeben Satzes von Injektionsparameters während einer Injektion umfaßt.

27. Angiographischer Injektor nach Anspruch 26, weiter gekennzeichnet durch eine Einrichtung (142) zum Voreinstellen eines Werts der Druckanstiegs-/Druckabfallszeit des genannten Kontrastmittels zwischen aufeinanderfolgenden Phasen einer Mehrphaseninjektionsfolge.

28. Angiographischer Injektor nach Anspruch 1, weiter gekennzeichnet durch eine selbsteichende Einrichtung zum Erzeugen eines Teststeuersignals, umd en genannten Antrieb auf eine Teststellung einzustellen, welches genannte Kontrollglied so wirkt, daß es die augenblockliche Stellung des Antriebs als Betriebsbedingung feststellt, eine Einrichtung zum Vergleichen der Teststellung mit der augenblicklichen Stellung und eine Einrichtung zum Nacheichen des genannten Antriebs auf der Grundlage des genannten Vergleichs.

29. Angiographischer Injektor nach Anspruch 1, weiter gekennzeichnet durch: eine selbsttätig arbeitende Prüfeinrichtung zum automatischen Prüfen der Genauigkeit der genannten Steuereinheit und zum Erzeugen einer Anzeige einer vorbestimmten Abweichung von einer verlangten Genauigkeit und in deren Folge zum Verhindern einer Injektion.

30. Angiographischer Injektor nach Anspruch 29, dadurch gekennzeichnet, daß die genannte selbsttätig arbeitende Prüfeinrichtung eine Einrichtung zum Prüfen der Betriebsgenauigkeit der genannten Steuereinheit und der genannten Speicher aufweist.

31. Angiographischer Injektor nach Anspruch 30, dadurch gekennzeichnet, daß die genannte selbsttätig arbeitende Prüfeinrichtung eine Einrichtung zum Ausführen von Befehlen der genannten Steuereinheit aufweist, wobei Testdaten und Testeinrichtungen zum Vergleichen der Ergebnisse jedes einzelnen der genannten ausgeführten Befehle mit einem vorher aufgestellten richtigen Ergebnis angewandt werden.

32. Angiographischer Injektor nach Anspruch 30, dadurch gekennzeichnet, daß die genannte selbsttätig arbeitende Prüfeinrichtung eine Einrichtung zum Einschreiben bekannter Daten in den genannten Speicher aufweist, ferner eine Einrichtung zum Abrufen der genannten, in den genannten Speicher eingeschriebenen bekannten Daten, und eine Einrichtung zum Vergleichen der abgerufenen Daten mit den bekannten Daten, um auf diese Weise den Betriebszustand des genannten Speichers zu überprüfen.

33. Angiographischer Injektor nach Anspruch 30, dadurch gekennzeichnet, daß der genannte Speicher einen ROM-Speicher aufweist und die genannte selbsttätig arbeitende Prüfeinrichtung eine Überprüfungseinrichtung zum Prüfen des Inhalts des genannten ROM-Speichers aufweist.

34. Angiographischer Injektor nach Anspruch 30, dadurch gekennzeichnet, daß die genannte selbsttätig arbeitende Prüfeinrichtung außerdem eine Analog/Digital- und Digital/Analog-Testeinrichtung zum Überprüfen der Genauigkeit von Sensoren, A/D-Umsetzern und D/A-Umsetzern des genannten Injektors aufweist.

35. Angiographischer Injektor nach Anspruch 30, dadurch gekennzeichnet, daß die genannte selbsttätig arbeitende Prüfeinrichtung ein in ein Analogsignal umzuwandelndes Digitalwort einem Digital/Analog-Umsetzer zuleitet, und gekennzeichnet durch eine Einrichtung zum Überprüfen der Genauigkeit des genannten Digital/Analog-Umsetzers aufgrund der übertragênen Information.

36. Angiographischer Injektor nach Anspruch 34, dadurch gekennzeichnet, daß die genannte selbsttätig arbeitende Prüfeinrichtung ein geeichtes Analogsignal erzeugt und einem A/D-Umsetzer zuleitet, und gekennzeichnet durch eine Einrichtung zum Überprüfen der Genauigkeit des von dem genannten A/D- Umsetzer umgesetzten Digitalsignals.

37. Angiographischer Injektor nach Anspruch 34, dadurch gekennzeichnet, daß die genannte selbsttätig arbeitende Prüfeinrichtung außerdem eine Einrichtung zum Prüfen der genannten Druckbegrenzungseinrichtung aufweist.

38. Angiographischer Injektor nach Anspruch 30, dadurch gekennzeichnet, daß die selbsttätig arbeitende Prüfeinrichtung außerdem eine Einrichtung zum Überwachen des an den genannten Antrieb gelieferten Stroms auf der Grundlage einer geeichten gesteuerten Steuerstromeinstellung aufweist, um auf diese Weise die Arbeitsweise der genannten Antriebseinrichtung zu überprüfen.

39. Angiographischer Injektor nach Anspruch 1, dadurch gekennzeichnet, daß die genannte Betriebsbedingung eine den Herzzyklus des Patienten wiedergebende EKG-Wellenform ist, und gekennzeichnet durch eine Einrichtung zur automatischen Vornahme einer Injektion einer gegebenen Menge Kontrastmittel in gesteuerter Durchflußmenge während eines gegebenen Zeitintervalls des genannten Herzzyklusses, um auf diese Weise die Röntgenphotographie synchron zu der genannten EKG-Wellenform zu ermöglichen.

40. Angiographischer Injektor nach Anspruch 1, mit einem ebenen, abgedichteten Membransteuerfeld (14), das mindestens einen Eingabeblock für jeden bzw. den genannten mindestens einen Injektionsparameter aufweist, wobei die genannten Eingabeblöcke zum Eingeben des genannten Injektionsparameters betätigt werden können.

41. Angiographischer Injektor nach Anspruch 1, umfassend ein abgedichtetes, ebenes Mebransteuerfeld (14) mit einem vom Menschen ablesbaren Formatteil für die Wiedergabe der Eingaben des Operateurs und zum Anzeigen der den Injektor betreffenden Ausgabeinformation, einen zweiteiligen Funktionskontroll- und -anzeigefeldteil zum Eingeben und zum Anzeigen von Injektionsparametern, ein Zahlentastenfeld (134) zum Eingeben von alphanumerischen Informationen, einen vorprogrammierten Injektionsteil zum Speichern von zuvor festgelegten Injektionsparametern, und einen Aktivierungssteuerteil (170) zum Einschalten des genannten Antriebs nach Maßgabe von in dem genannten Injektor gespeicherten Informationen und der Eingabe des Operateurs.

42. Angiographischer Injektor nach Anspruch 41, dadurch gekennzeichnet, daß der genannte zweiteilige Funktionssteuer- und Anzeigefeldteil eine Einrichtung zum Löschen des genannten Wiedergabefeldteils und des genannten Formatteils für vom Menschen ablesbaren Informationen umfaßt.

43. Angiographischer Injektor nach Anspruch 41, weiter gekennzeichnet durch einen Steuerteil (182) für Mehrphaseninjektion für die Aufnahme von mindestens zwei Sätzen von Injektionssteuerparametern, und eine Einrichtung zum Vornehmen von Injektionen nacheinander nach Maßgabe jedes Satzes von Injektionssteuerparametern.

44. Angiographischer Injektor nach Anspruch 41, dadurch gekennzeichnet, daß die genannte Steuereinheit eine Einrichtung aufweist, die den Operateur veranlaßt, eine Anzahl Injektionsparameter anzugeben, und daß der genannte zweiteilige Funktionssteuer- und Wiedergabefeldteil eine Leuchtanzeige in Zuordnung zu jedem der genannten Injektionsparameter aufweist, sowie eine während der Angabe jedes der genannten Eingabeparameter wirksame Einrichtung zum Herbeiführen des Aufleuchtens der genannten Leuchtanzeige, um den Operateur zum Eingeben eines bestimmten Injektionsparameters zu veranlassen, und eine Einrichtung zum Beenden der Leuchtanzeige, wenn der Injektionsparameters eingegeben worden ist, und zum Herbeiführen des Aufleuchtens einer nächstfolgenden Leuchtanzeige, die einem nächstfolgenden, von dem Operateur einzugebenden Eingabeparameter zugeordnet ist.

45. Angiographischer Injektor nach Anspruch 44, dadurch gekennzeichnet, daß die genannte Steuereinheit außerdem eine Einrichtung (76) zum Feststellen von Fehlern bei einem von einem Operateur eingegebenen Injektionsparameter sowie eine Einrichtung zum Erzeugen eines Schallsignals bei Feststellung des genannten Fehlers aufweist.

46. Angiographischer Injektor nach Anspruch 1, dadurch gekennzeichnet, daß der genannte Behälter eine Spritze mit einer Mehrzahl Skalen (228, 230, 232) an einer Seitenwand der Spritze sowie eine Leuchtanzeige (226) zum Bezeichnen einer gewählten Skala aufweist, wodurch eine visuelle Überwachung des während eines Injektionsvorgangs injizierten Volumens möglich wird.

47. Angiographischer Injektor nach Anspruch 1, dadurch gekennzeichnet, daß die genannte Steuereinheit eine Einrichtung (182) zum Herbeiführen von Mehrphasen-Injektionsfolgen aufweist, worin während jeder Injektionsphase das Injizieren von Kontrastmittel gemäß den einzelnen Sätzen von Injektionsparametern Überwacht wird, die der Steuereinheit nacheinander zugeführt werden.

48. Angiographischer Injektor nach Anspruch 47, dadurch gekennzeichnet, daß zwischen jeder Phase der genannten Mehrphaseninjektion die Größe des Druck-Anstiegs-/Abfalls des Kontrastmittels auf einen vorgegebenen Pegel gesteuert wird.

49. Angiographischer Injektor nach Anspruch 48, weiter gekennzeichnet durch eine Einrichtung zum Überprüfen jedes Parameters eines Satzes von Injektionsparametern zwischen aufeinanderfolgenden Phasen einer Mehrphasen-Injektionsfolge.

50. Angiographischer Injektor nach Anspruch 1, weiter gekennzeichnet durch:

eine Steuereinheit zum Regeln der Abgabe des genannten Kontrastmittels, welche Steuereinheit außerdem eine selbsttätig arbeitende Prüfeinrichtung zum Überwachen eines Betriebszustands des genannten angiographischen Injektors und zum Erzeugen eines Fehlersignals beim Erkennen einer Störung aufweist.

51. Angiographischer Injektor nach Anspruch 50, umfassend Speicher (18) zum Speichern von die genannte Injektion betreffenden Informationen und A/D- und D/A-Umsetzer zum Anschließen von analogen und digitalen Steuer- und Zustandssignalen, wobei der genannte überwachte Zustand den Betriebszustand der genannten Steuereinheit, des genannten Speichers und der genannten A/D- und D/A-Umsetzer umfaßt.

52. Angiographischer Injektor nach Anspruch 50, dadurch gekennzeichnet, daß der genannte überwachte Zustand umfaßt: übermäßig hohen Druck des genannten Kontrastmittels während einer Injektion, von dem genannten Antrieb aufgenommener übermäßig hoher Strom, übermäßig großer Positionierungsfehler des genannten Antriebs, und wobei die genannte Steuereinheit dafür sorgt, daß der genannte Injektor gesperrt wird, wenn die genannten Betriebszustände übermäßige Abweichungen zeigen.

53. Angiographischer Injektor nach Anspruch 1, dadurch gekennzeichnet, daß die genannte Steuereinheit außerdem eine Computerschnittstelle (20) zum Herstellen externer Kommunikation mit dem genannten angiographischen Injektor umfaßt.

54. Angiographischer Injektor nach Anspruch 53, dadurch gekennzeichnet, daß die genannte Computerschnittstelle einen Datenkanal zur Herstellung externer Kommunikation mit dem genannten externen Gerät aufweist, um Informationen, einschließlich Steuer- und Zustandsinformation, zu übertragen.

55. Angiographischer Injektor nach Anspruch 54, dadurch gekennzeichnet, daß die genannte externe Computerschnittstelle einen Universal-Asynchron-Empfangs/Sende-Port aufweist.

**Revendications**

1. Injecteur angiographique pour injecter un moyen de contraste dans le système vasculaire d'un patient, comportant:

des moyens (204) pour tenir un réservoir contenant le moyen de contraste et coportant un orifice de décharge par lequel le moyen est délivré, et

des moyens d'entraînement (24) pour effectuer la décharge dudit moyen à travers l'orifice de décharge, caractérisé en ce que l'injecteur comporte par ailleurs:

des moyens de réception pour recevoir au moins un paramètre de commande de l'injection qui comporte au moins un paramètre sélectionné parmi le groupe de paramètres suivants: le débit du moyen de contraste, le volume du moyen de contraste, la durée d'une injection, la limite de pression dudit moyen de contraste, le retard de la radiographie, le retard de l'injection et la durée de l'augmentation/la diminution de la pression du flux du moyen de contraste,

une unité de commande (10) pour générer au moins un signal de commande de l'injection, en fonction d'au moins un paramètre d'injection transmis, et pour transmettre ledit signal de commande auxdits moyens d'entraînement (24) afin de réaliser une décharge déterminée dudit moyen,

dans lequel lesdits moyens d'entraînement (24) et l'unité de commande (10) comportent un système servo à boucle fermée qui réagit à un signal de commande de la position produit par ladite unité de commande (10).

2. Injecteur angiographique selon la revendication 1, dans lequel ledit paramètre de commande de l'injection est la pression dudit moyen dans le réservoir et dans lequel lesdits moyens d'entraînement (24) et l'unité de commande (10) comportent des moyens de limitation de la pression (60) qui réagissent à un signal de la pression limite préétablie pour empêcher la réaction dudit système servo audit signal de commande de la position lorsque la pression dans ledit réservoir dépasse ladite limite pré-établie.

3. Injecteur angiographique selon la revendication 2, dans lequel lesdits moyens de limitation de la pression comportent des moyens pour recevoir le signal de limitation de la pression pré-établie dans des unités de PSI, KPA, KG ou ATU et dans lequel ledit injecteur angiographique comporte, par ailleurs, des moyens de commutation pour sélectionner l'une desdites unités indicatives du signal de limitation de la pression pré-établie.

4. Injecteur angiographique selon la revendication 1 ou 2, dans lequel le réservoir comporte une seringue ayant un piston (202) pour forcer le moyen à travers l'orifice de décharge de celle-ci, lesdits moyens d'entraînement comportant un servo moteur à courant continu (40) dont l'action est associée audit piston, et ladite unité de commande comporte des moyens pour fournir audit moteur à courant continu un courant continu (52) dérivé d'un courant propulseur à largeur d'impulsion modulée ayant une durée d'enclenchement qui est proportionnelle à la différence entre la position effective et la position souhaitée dudit piston pendant une injection.

5. Injecteur angiographique selon les revendications 1 ou 4, comportant par ailleurs un moyen d'arrêt mécanique (26) qui réagit à une limite avant pré-établie dudit piston pour empêcher lesdits moyens d'entraînement de délivrer le moyen de contraste à partir de la seringue, ladite limite avant étant déterminée par ladite unité de commande.

6. Injecteur angiographique selon la revendication 5, dans lequel ledit moyen d'arrêt mécanique comporte un élément de blocage (212) et un moteur d'arrêt à courant continu (214) dont l'action est associée audit élément de blocage, des moyens (66) pour la surveillance de la position dudit piston, des moyens (74) pour produire un signal d'arrêt lorsque la position dudit piston atteint la dite limite avant et des moyens réagissant audit signal d'arrêt pour actionner ledit moteur d'arrêt qui, à son tour, avance ledit élément de blocage pour empêcher mécaniquement le mouvement avant du piston.

7. Injecteur angiographique selon la revendication 6, dans lequel l'unité de commande comporte des moyens (110) pour régler ladite limite avant pré-établie dudit élément de blocage en fonction de la valeur d'un paramètre d'injection qui lui est fourni.

8. Injecteur angiographique selon la revendication 7, comportant par ailleurs des moyens de relais de sécurité (28, 44) qui actionnent en alternance, soit les moyens d'entraînement, soit ledit régulateur de blocage mécanique, pour empêcher le mouvement desdits moyens d'entraînement pendant que le régulateur de blocage mécanique est actionné.

9. Injecteur angiographique selon la revendication 8, comportant par ailleurs un témoin lumineux pour visualiser la position dudit élément de blocage mécanique et un deuxième témoin lumineux correspondant à la position dudit piston par rapport audit premier témoin lumineux.

10. Injecteur angiographique selon la revendication 1, comportant par ailleurs:

des moyens de surveillance (30) pour empêcher les moyens d'entraînement de réagir en cas de panne de ladite unité de commande.

11. Injecteur angiographique selon la revendication 10, dans lequel lesdits moyens de surveillance comportent un chronomètre pouvant être remis à zéro, réagissant à un signal de réglage pour produire un signal d'arrêt après un intervalle de temps prédéterminé, et ladite unité de commande qui comporte des moyens pour produire périodiquement ledit signal de remise à zéro, bloquant passivement lesdits moyens d'entraînement en cas de panne de ladite unité de commande.

12. Injecteur angiographique selon les revendications 1 ou 11, dans lequel ladite unité de commande comporte des moyens pour sélectionner au moins un paramètre d'injection par un opérateur dudit injecteur, facilitant ainsi l'usage de celui-ci.

13. Injecteur angiographique selon la revendication 12, dans lequel ledit paramètre d'injection unique au moins comporte au moins un paramètre sélectionné parmi un groupe de paramètres: le débit du moyen de contraste, le volume du moyen de contraste, la durée d'une injection, la limite de pression dudit moyen de contraste, le retard de la radiographie, le retard d'injection et la durée de l'augmentation et de la chute de la pression du moyen de contraste.

14. Injecteur angiographique selon la revendication 1 ou 13, comportant des moyens de réception ayant un panneau de commande scellé, plat, à membrane (14), avec des blocs d'entrée individuels associés à chacun desdits paramètres d'injection, au moins d'un, chacun desdits blocs d'entrée pouvant être opéré pour entrer un paramètre d'injection associé dans ladite unité de commande.

15. Injecteur angiographique selon la revendication 14, dans lequel ladite unité de commande comporte, par ailleurs, des moyens d'activation (170) réagissant à la commande de chacun desdits blocs d'entrée, au moins d'un, pour enclencher lesdits moyens d'entraînement et pour les bloquer lorsqu'au moins un quelconque desdits blocs d'entrée n'est pas actionné.

16. Injecteur angiographique selon les revendications 1 ou 15, comportant par ailleurs une première mémoire (18) pour mémoriser au moins un paramètre d'injection, une mémoire secondaire pour mémoriser un double du contenu dudit au moins un paramètre d'injection, des moyens de vérification du paramètre pour comparer les paramètres d'injection desdites mémoires première et secondaire, et pour bloquer lesdits moyens d'entraînement lorsqu'il y a un désaccord entre les paramètres d'injection.

17. Injecteur angiographique selon la revendication 1 ou 13, comportant par ailleurs une mémoire (18) pour mémoriser un ensemble de paramètres d'injection, des moyens pour produire une étiquette d'identification pour chaque ensemble desdits au moins un paramètre d'injection et pour mémoriser ladite étiquette avec ledit ensemble dans ladite mémoire, des moyens de rappel réagissant à une certaine étiquette pour rappeler de ladite mémoire un ensemble associé de paramètres d'injection et pour fournir ledit ensemble de paramètres d'injection auxdits moyens de réception, permettant ainsi le rappel rapide d'ensemble de paramètres d'injection de routine, sans que l'opérateur ait à faire des entrées préalables à l'injection.

18. Injecteur angiographique selon la revendication 17, comportant par ailleurs des moyens pour rappeler et traverser en séquence chacun desdits paramètres d'injection rappelés d'un ensemble donné et pour tirer une information afin de modifier un paramètre d'injection particulier.

19. Injecteur angiographique selon la revendication 17, comportant par ailleurs des moyens pour vérifier chacun desdits paramètres d'entrée rappelés avant une injection.

20. Injecteur angiographique selon la revendication 19, dans lequel lesdits moyens de vérification comportent un commutateur manuel associé à chacun desdits, au moins un, paramètres d'injection.

21. Injecteur angiographique selon la revendication 19, dans lequel lesdits moyens de vérification comportent des mémoires redondantes pour la mémorisation de chacun desdits paramètres d'injection, des moyens pour comparer le contenu dudit paramètre d'injection stocké dans lesdites mémoires redondantes et des moyens pour mettre hors service ledit injecteur ou pour alerter l'opérateur au cas où une différence apparaît lors de la comparaison des données entre les dites mémoires redondantes.

22. Injecteur angiographique selon la revendication 21, dans lequel chacune desdites mémoires redondantes comporte une mémoire d'accès randomisée, alimentée par une pile à part.

23. Injecteur angiographique selon la revendication 16, dans lequel lesdites mémoires première et secondaire comportant des moyens pour conserver lesdits paramètres d'injection en absence de courant d'alimentation dudit injecteur.

24. Injecteur angiographique selon la revendication 1, comportant par ailleurs: un d'interface (20) connecté à ladite unité de commande pour le transfert d'information d'état ou d'un paramètre d'injection audit injecteur.

25. Injecteur angiographique selon la revendication 24, dans lequel ledit interface comporte une porte asynchrone de réception/émission pour établir la communication avec un dispositif externe.

26. Injecteur angiographique selon la revendication 1, dans lequel ladite unité de commande comporte une commande d'injection multiphases pour le changement séquentiel d'un ensemble donné de paramètres d'injection pendant une injection.

27. Injecteur angiographique selon la revendication 26, comportant par ailleurs des moyens (142) pour un pré-établir une valeur de durée d'augmentation/de diminution de la pression dudit moyen de contraste dans les phases successives d'une séquence d'injection multi-phases.

28. Injecteur angiographique selon la revendication 1, comportant par ailleurs des moyens d'autocalibrage pour produire un signal de commande de test afin de positionner lesdits moyens d'entraînement dans une position de test, lesdits moyens de contrôle ayant la fonction de relever la position instantanée des moyens d'entraînement comme condition opérationnelle, des moyens pour comparer la psition de test avec la position instantanée et des moyens pour recalibrer lesdits moyens d'entraînement sur la base de ladite comparaison.

29. Injecteur angiographique selon la revendication 1, comportant par ailleurs des moyens de contrôle pour la vérification automatique de la précision de ladite unité de commande et pour produire une indication d'un écart prédéterminé par rapport à la précision désirée afin d'empêcher une injection en réaction à l'écart.

30. Injecteur angiographique selon la revendication 29, dans lequel les moyens de contrôle automatique comportent des moyens pour vérifier les conditions opérationnelles de ladite unité de commande et desdites mémoires.

31. Injecteur angiographique selon la revendication 30, dans lequel les moyens de contrôle automatique comportent des moyens pour exécuter les ordres de ladite unité de commande en utilisant les données et les moyens de contrôle pour vérifier les résultats de chacun des ordres exécutés avec un résultat correct pré-établi.

32. Injecteur angiographique selon la revendication 30, dans lequel les moyens de contrôle automatique comportent des moyens pour écrire les données conues dans lesdites mémoires, des moyens pour relire lesdites données écrites dans les mémoires et des moyens de comparaison des données relues et les données connues, pour vérifier l'état opérationnel desdites mémoires.

33. Injecteur angiographique selon la revendication 30, dans lequel les mémoires comportent une mémoire ROM et dans lequel lesdits moyens de contrôle automatique comportent des moyens de vérification pour vérifier le contenu de ladite mémoire ROM.

34. Injecteur angiographique selon la revendication 30, dans lequel les moyens de contrôle automatique comportent par ailleurs des moyens de test analogiques/digitaux et digitaux/analogiques pour vérifier la précision de capteurs, de convertisseurs A/D et de convertisseurs D/A dudit injecteur.

35. Injecteur angiographique selon la revendication 30, dans lequel les moyens de contrôle automatique envoient un mot digital pour être converti dans un signal analogique à un convertisseur digital/analogique et dans lequel des moyens vérifiant la précision dudit convertisseur digital/analogique sur la base de ladite information transmise.

36. Injecteur angiographique selon la revendication 34, dans lequel lesdits moyens de contrôle automatique génèrent et transmettent un signal analogique calibré à un convertisseur A/D et dans lequel des moyens vérifient la précision du signal digital converti par ledit convertisseur A/D.

37. Injecteur angiographique selon la revendication 34, dans lequel lesdits moyens de contrôle automatique comportent, par ailleurs, des moyens pour la vérification desdits moyens de limitation de la pression.

38. Injecteur angiographique selon la revendication 30, dans lequel les moyens de contrôle automatique comportent, par ailleurs, des moyens pour la surveillance du courant fourni auxdits moyens d'entraînement sur la base d'un réglage de courant de commande calibré et contrôlé pour vérifier le fonctionnement desdits moyens d'entraînement.

39. Injecteur angiographique selon la revendication 1, dans lequel ladite condition de fonctionnement surveillée est une forme d'onde ECG représentant un cycle cardiaque d'un patient, et dans lequel des moyens effectuent automatiquement l'injection d'une quantité donnée de moyen de contraste à un débit contrôlé pendant un intervalle déterminé dudit cycle cardiaque pour faciliter ainsi la synchronisation de la radiographie avec ladite forme d'onde ECG.

40. Injecteur angiographique selon la revendication 1, comportant un panneau (14) de commande scellé, plat, à membrane, ayant au moins un dispositif d'entrée pour chaque ou au moins ledit paramètre d'injection, lesdits dispositifs d'entrée pouvant être opérés pour entrer ledit paramètre d'injection.

41. Injecteur angiographique selon la revendication 1, comportant un peanneau (14) de commande scellé, plat, à membrane, ayant une section d'un format pouvant être les par un être humain pour afficher les entrées de l'opérateur et pour indiquer l'information de sortie concernant l'injecteur, un panneau à double fonction de contrôle et d'affichage pour l'entrée et l'affichage de paramètres d'injection, une partie à touches numériques (134) pour entrer une information alphanumérique, une section d'injection pré-programmée pour la mémorisation de paramètres d'injection pré-établis et une section de contrôle de l'activation (170) pour permettre auxdits moyens d'entraînement de répondre aux informations mémorisées dans ledit injecteur et à l'entrée de l'opérateur.

42. Injecteur angiographique selon la revendication 41, dans lequel la section de panneau à double fonction de contrôle et d'affichage comporte des moyens pour effacer l'information dans ladite section de panneau d'affichage et la section à format pouvant être lue par un être humain.

43. Injecteur angiographique selon la revendication 41, comportant par ailleurs une section de commande de l'injection multi-phases (182) pour recevoir au moins deux ensembles de paramètres de commande de l'injection, et des moyens pour effectuer successivement l'injection, conformément à chaque ensemble de paramètres de commande de l'injection.

44. Injecteur angiographique selon la revendication 41, dans lequel l'unité de commande comporte des moyens pour obtenir d'un opérateur un nombre de paramètres d'injection et dans lequel ladite section à double fonction de commande et d'affichage comporte un témoin lumineux associé à chacun desdits paramètres d'injection, des moyens pouvant être opérés pendant l'obtention de chaque dit paramètre d'entrée pour allumer brièvement ledit témoin lumineux afin d'avertir l'opérateur qu'un paramètre d'injection particulier doit être entré, et des moyens pour éteindre le témoin lorsque le paramètre d'injection a été entré et pour allumer brièvement un témoin lumineux successif associé à un paramètre d'entrée successif qui doit être entré par l'opérateur.

45. Injecteur angiographique selon la revendication 44, dans lequel ladite unité de commande comporte par ailleurs des moyens (76) pour reconnaître des erreurs dans le paramètre d'injection entré par un opérateur et des moyens générant un signal d'alarme acoustique après la détection de ladite erreur.

46. Injecteur angiographique selon la revendication 1, dans lequel le réservoir comporte une seringue ayant une pluralité d'échelles (228, 230, 232) disposées sur la paroi latérale de la seringue, et un témoin lumineux (226) pour identifier une échelle sélectionnée permettant l'observation visuelle du volume injecté pendant la procédure d'injection.

47. Injecteur angiographique selon la revendication 1, dans lequel ladite unité de commande comporte des moyens (182) pour effectuer des séquences d'injection multi-phases et dans lesquelles chaque phase d'injection du moyen de contraste est surveillée d'après les ensembles individuels de paramètres d'injection successivement fournis à ladite unité de commande.

48. Injecteur angiographique selon la revendication 47, dans lequel l'importance de l'augmentation/la chute de la pression entre chaque phase de ladite injection multi-phases du moyen de contraste est réglée à un niveau prédéterminé.

49. Injecteur angiographique selon la revendication 48, comportant par ailleurs des moyens pour vérifier chaque paramètre d'un ensemble de paramètres d'injection entre les phases successives d'une séquence d'injection multi-phases.

50. Injecteur angiographique selon la revendication 1, comportant par ailleurs:

une unité de commande pour régler la décharge dudit moyen de contraste, ladite unité de commande comportant, par ailleurs, des moyens de contrôle automatique pour la surveillance de l'état opérationnel dudit injecteur angiographique et pour produire un signal d'erreur au cas où un défaut est détecté.

51. Injecteur angiographique selon la revendication 50, comportant par ailleurs des mémoires (18) pour mémoriser l'information concernant ladite injection et des convertisseurs A/D et D/A pour le raccordement de signaux de commande anglogiques et digitaux et de signaux d'état, ladite condition surveillée comportant l'état opérationnel de ladite unité de commande de ladite mémoire et des convertisseurs A/D et D/A.

52. Injecteur angiographique selon la revendication 50, dans lequel ladite condition surveillée comporte la pression excessive du moyen de contraste pendant une injection, un courant excessif consommé par lesdits moyens d'entraînement, une erreur de position excessive desdits moyens d'entraînement, et dans lequel ladite unité de commande réagit pour bloquer ledit injecteur au cas où lesdits états opérationnels sont excessifs.

53. Injecteur angiographique selon la revendication 1, dans lequel ladite unité de commande comporte par ailleurs un interface d'ordinateur (20) pour constituer une communication externe avec ledit injecteur angiographique.

54. Injecteur angiographique selon la revendication 53, dans lequel ledit interface d'ordinateur comporte un canal de données pour permettre la communication externe avec ledit dispositif externe afin de transférer des informations, y compris l'information de commande et d'état.

55. Injecteur angiographique selon la revendication 54, dans lequel ledit interface d'ordinateur externe comporte une porte universelle asynchrone de réception/émission.

FIG. 1A
18
STORAGE MODULE
BATT.
20
COMPUTER INTERFACE
DATA
DATA
EXTERNAL COMPUTER
24
SERVO AMP.
PLUNGER POSITION ERROR
PRESSURE LIMIT COMMAND
ACTUAL PLUNGER POSITION
PRESSURE LIMIT INDICATION
64
22
SERVO CONTROL
10
CPU
DATA / CONTROL
INTERRUPT
OPEN RELAYS
30
WATCHDOG CKT
80
ARM
SAFETY RELAY
+V
+ MOTOR DC
LOW VOLTAGE DC
28
SWITCHES
FWD
REV
MECH. STOP POSITION COMMAND
MECH. STOP POSITION
26
MECH. STOP CONTROLLER
12
I/O MODULE
DATA
DISPLAYS / LIGHTS
SWITCH CLOSURES
14
FRONT PANEL
15
NUMERIC DISPLAYS
16
KEYBOARD
112
START
EXT. START
114

FIG. 2
42
MOTOR DC +
(VIA SAFETY RELAY)
22
SERVO CONTROL
PLUNGER POSITION ERROR
54
50
ERROR DETECT CKT.
FWD DRIVE
REVERSE DRIVE
46
A
56
B
DUTY CYCLE CONTROL
52
PULSE WIDTH MODULATION CONTROL CKT.
REVERSE DRIVE
FWD DRIVE
58
C
40
PLUNGER MOTOR
INJECTOR HEAD
48
D
DUTY CYCLE CONTROL
62
PRESSURE LIMIT COMMAND
PRESSURE LIMIT CKT.
60
SAMPLED MOTOR CURRENT
ACTUAL PLUNGER POSITION
68
PLUNGER POSITION CKT.
66
64
PRESSURE LIMIT INDICATION
SERVO AMP.
24

72
74
DATA BUS
70
BIDIRECTIONAL BUFFER
CONTROL D/A'S
62
PRESSURE LIMIT COMMAND
MECH. STOP POSITION COMMAND
75
ANALOG MULTIPLEXER
PLUNGER POSITION COMMAND
A/D
77
78
SELECT
PERIPHERAL INPUT / OUTPUT CKT.
PLUNGER DESIRED POSITION
(MANUAL HEAD SWITCHES)
FWD
REV
76
54
PLUNGER POSITION ERROR
68
ERROR SIGNAL CKT
ACTUAL PLUNGER POSITION
MECH. STOP DIRECTION CONTROL
ACTUAL MECH. STOP POSITION
108
PRESSURE LIMIT INDICATION (P.S.I.)
64
ARM
80
SERVO CONTROL
22

FIG. 3

FIG. 1B
10
CPU
NMI
ADDRESS
13
DATA SELECTOR
STROBE
11
RETRIGGERABLE TIMER
TO SAFE RELAY
FIG. 4
MECH. STOP CONTROLLER
LOW VOLTAGE DC
94
DIRECTION CKT.
109
100
102
104
106
90
M.S. MOTOR
INJECTOR HEAD
110
MECH. STOP POSITION CKT.
MECH. STOP POSITION POTENTIOMETER (FROM HEAD)
111
108
MECH STOP DIRECTION CONTROL
ACTUAL POSITION OF MECH STOP

FIG. 5A
FIG. 5B
200
202
204
206
208
210
212
214
216
218
220
222
224
226
228
230
232
234
236
238
240
242

SINGLE
MULTI
DISARM
MULTI
SINGLE
ARMING
TOTAL LEVELS
LEVEL
NEW PATIENT/ START OVER
PROGRAM
RECALL
STORE
ABC 7
DEF 8
GHI 9
JKL 4
MNO 5
PQR 6
STU 1
VWX 2
YZ/ 3
SHIFT + STATUS
0 YES
• NO
SENTINEL
INJECTION DURATION
SET
ACTUAL
DURATION
X-RAY
INJECTOR
DELAY
SECONDS
FLOW RATE
SET
ACTUAL
ML/SET
ML/MIN
ML/HR
PRESSURE
SET
ACTUAL
PSI
KG
ATU
KPA
RISE/FALL
SECONDS
VOLUME
SET
ACTUAL
ML
FRONT PANEL 14
FIG. 6
14
130
132
134
136
138
142
144
146
148
150
152
154
156
158
160
162
164
166
168
170
172
174
176
178
180
181
182
184
186

POWER UP: BEGIN SELF TESTS.
TEST CPU INSTRUCTION SET.
SENTINEL: "INSTRUCTION TEST IN PROGRESS."
ALL TESTS PASSED?
NO
IDENTIFY FAILING INSTRUCTION AND DISPLAY SENTINEL MESSAGE.
STOP
YES
WRITE DATA PATTERNS INTO RAMS.
READ DATA BACK AND COMPARE WITH EXPECTED PATTERNS.
PATTERNS MATCH?
NO
IDENTIFY FAILING ADDRESS, DATA VALUES & DISPLAY SENTINEL MESSAGE.
STOP
YES
COMPUTE CHECKSUM OF RAM.
READ STORED CHECKSUM FROM RAM LOCATION.

FIG 7A

CHECKSUMS MATCH ?
NO
SENTINEL : "ROM ERROR.
STOP
YES
READ PRE-PROGRAMMED MEMORY.
READ BACK-UP MEMORY.
AGREE?
NO
GENERATE NON-MASKABLE INTERRUPT AND SENTINEL MESSAGE.
STOP
YES
COMPUTE CHECKSUM OF PRE-PROGRAMMED MEMORY.
READ STORED CHECKSUM FROM PRE-PROGRAMMED MEMORY.
AGREE?
NO
SENTINEL: "PRE-PROGRAMMED MEMORY ERROR.
STOP
YES
LOAD PATTERNS INTO D/A CONVERTERS.

FIG. 7B

TRANSFER ANALOG VALUES THROUGH MIXER TO A/D.
READ A/D VALUES.
DIGITAL VALUES MATCH?
NO
IDENTIFY FAILING DATA PATH WITH SENTINEL MESSAGE.
STOP
YES
CHECK VOLTAGES FROM MECHANICAL STOP AND PLUNGER POTS.
OPEN OR SHORT CIRCUIT?
YES
IDENTIFY FAILURE WITH SENTINEL MESSAGE.
STOP
NO
1
SENTINEL: "READY."
2
INJECTION IN PROGRESS?
NO
YES
SAMPLE ACTUAL PLUNGER POSITION.

FIG 7C

COMPUTE POSITION OF PLUNGER.
AGREE?
NO
3
OPEN SAFETY RELAY AND STOP INJECTION. DISPLAY MESSAGE.
STOP
YES
PRESSURE FLOW W/IN LIMITS?
NO
YES
WATCHDOG TIMEOUT?
NO
YES
IDENTIFY FAILURE.
STOP

FIG. 7D

POWER - UP : ALL DISPLAYS CLEARED TO ZERO.
ALL BACKLIGHTS OFF.
TOTAL LEVELS = 1, LEVEL = 1.
SENTINEL : "READY"
C D E F H J K L M
A
RISE/FALL BUTTON PRESSED?
YES
RISE/FALL LIGHT FLASHES
NO
ENTER 0.0 TO 9.9 SECONDS
1
FLOW RATE BUTTON PRESSED?
YES
RISE/FALL LIGHT STAYS ON
"SET" FLASHES. ENTER 0.0 TO 9.9 SECONDS
NO
2
NO
FLOW UNITS BUTTON PRESSED?
YES
LIGHT ML/SEC. OR ML/MIN. OR ML/HR.
DURATION BUTTON PRESSED?
NO
YES
FLOW RATE LIGHT STAYS ON. DURATION "SET" FLASHES. ENTER 0.0 TO 999.9 SECONDS
CALCULATE VOLUME = RATE X DURATION. DISPLAY UNDER "VOLUME"
3


FIG. 8A

VOLUME BUTTON PRESSED?
NO
YES
DURATION LIGHT STAYS ON. VOLUME "SET" FLASHES. ENTER 0.0 TO 999.9 ML/TIME.
CALCULATE DURATION = VOLUME/RATE AND DISPLAY.
4
PRESSURE BUTTON PRESSED?
YES
NO
VOLUME LIGHT STAYS ON. PRESSURE "SET" FLASHES. ENTER 0.0 TO 9999 PSI, , , OR
5
DELAY BUTTON PRESSED?
YES
PRESSURE LIGHT STAYS ON. DELAY "SET" FLASHES ENTER 0.0 TO 999.9 SECONDS.
3
SINGLE OR MULTIPLE ARM BUTTON PRESSED?
YES
NO
6

FIG. 8B

FIG. 8C
STORE BUTTON PRESSED?
YES
NO
SENTINEL: "ENTER PREPROGRAMMED INJECTION NUMBER.
ENTER PROGRAM NUMBER 1 TO 99 AND STORE INJECTION PARAMETERS FOR ALL LEVELS.
SENTINEL: "ENTER TITLE FIELD AND STORE."
ENTER TITLE FIELD (1 TO 32 CHARACTERS) AND STORE.
9
LEVEL ADVANCE?
YES
NO
DISPLAY NEXT LEVEL NUMBER.
E TO A
LEVEL DOWN?
YES
NO
DISPLAY PREVIOUS LEVEL IF ≥ 1.
F TO A
"NEW" PATIENT BUTTON PRESSED?
YES
ALL PARAMETERS ENTERED?
NO
YES
SENTINEL: "ENTER MISSING PARAMETER."
CHECK FOR ERRORS IN DATA ENTRIES. DISPLAY ERROR MESSAGES.
INSUFFICIENT VOLUME REMAINING.
FLOW RATE TOO HIGH.
PRESSURE TOO HIGH.
ANY ERRORS?
YES
NO
C TO A
MOVE MECHANICAL STOP TO OLD POSITION PLUS INJECT VOLUME.
FLASH "SINGLE" OR "MULTI" ARM LIGHT.
DISABLE MECHANICAL STOP. ENABLE PLUNGER DRIVE.
7
START SWITCH PRESSED?
NO

NO
SENTINEL: "NEW PATIENT. CUMULATIVE VOLUME = 0.
H TO A
YES
8
START INJECTION. "SINGLE" OR "MULTI" LIGHT STAYS ON.
"RECALL" BUTTON PRESSED?
NO
YES
ANY "ACTUAL" BUTTON PRESSED?
SENTINEL: "ENTER PROGRAM NUMBER TO RECALL."
INJECTION COMPLETE OR RELEASED START SWITCH?
YES
YES
ENTER PROGRAM NUMBER AND DISPLAY PARAMETERS AND TITLE FIELD.
NO
DISPLAY ACTUAL RISE/FALL, FLOW, DURATION, VOLUME, PRESSURE.
NO
FINAL LEVEL REACHED?
NO
"STATUS" BUTTON PRESSED?
K TO A
SENTINEL: "VERIFY ALL PARAMETERS BY PUSHING SET BUTTONS."
ADVANCE TO NEXT LEVEL.
YES
NO
YES
L TO A
J TO A
TURN ON "DISARM LIGHT. DISABLE PLUNGER DRIVE.
DISPLAY ALL ACTUAL FOR SELECTED LEVEL.
D TO A
M TO A
FIG. 8D